Europäisches Patentamt

(19) European Patent Office

Office européen des brevets

(11) EP 0 336 379 B1

## (12) EUROPEAN PATENT SPECIFICATION

(45) Date of publication and mention
of the grant of the patent:
**07.02.1996 Bulletin 1996/06**

(51) Int. Cl.$^6$: **A61K 39/395**, A61K 39/42,
A61K 39/39

(21) Application number: 89105907.3

(22) Date of filing: **04.04.1989**

(54) **Antibody heteroconjugates for use in regulation of lymphocyte activity**

Antikörper-Heterokonjugate zur Regulation der Lymphozytenaktivität

Hétéro-conjugués d'anticorps pour l'utilisation dans la régulation de l'activité de lymphocytes

(84) Designated Contracting States:
**AT BE CH DE ES FR GB GR IT LI LU NL SE**

(30) Priority: **04.04.1988 US 176825**
**14.11.1988 US 271934**

(43) Date of publication of application:
**11.10.1989 Bulletin 1989/41**

(73) Proprietor: **ONCOGEN LIMITED PARTNERSHIP**
**Seattle, WA 98121 (US)**

(72) Inventor: **Ledbetter, Jeffrey A.**
**Seattle, WA (US)**

(74) Representative: **Kinzebach, Werner, Dr. et al**
**D-81633 München (DE)**

(56) References cited:
**EP-A- 0 153 871** **EP-A- 0 235 805**
**EP-A- 0 241 907** **WO-A-85/04811**
**US-A- 4 381 292**

• **CHEMICAL ABSTRACTS, vol. 106, no. 5, 02
February 1987, Columbus, OH (US); D.M. SEGAL
et al., p. 371, no. 30993a/**
• **CHEMICAL ABSTRACTS, vol. 104, no. 13, 31
March 31 1986, Columbus, OH (US); M.A. LIU et
al., p. 549, no. 107576k/**

**Description**

TECHNICAL FIELD OF THE INVENTION

The present invention relates to novel antibody heteroconjugates and their use in the regulation of the activity of lymphocytes such as T cells and B cells. More particularly, the invention relates to heteroconjugates comprised of at least two antibodies coupled to each other, each antibody being reactive with a different lymphocyte surface antigen. These heteroconjugates appear to act by binding to lymphocytes and bringing the respective cell surface antigens they react with into physical proximity to each other, resulting in enhanced or reduced signal transduction and proliferation of lymphocytes. According to a preferred embodiment of the invention, an antibody reactive with the T cell receptor or its associated CD3 complex is cross-linked to an antibody reactive with a second T cell surface antigen, such as CD2, CD4, CD6 or CD8 to enhance activation and function of T cells. According to another preferred embodiment, an antibody reactive with the CD5 T cell surface antigen is cross-linked to an antibody reactive with a second T cell surface antigen, such as CD4, CD6 or CD8 to enhance lymphocyte activation and function. In an additional preferred embodiment, an antibody reactive with the CD45 family of antigens is cross-linked with an antibody reactive with a second T cell surface antigen such as CD2, CD3, CD5 or CD28 to inhibit lymphocyte activation and function. In yet another preferred embodiment, an antibody reactive with the CD4 antigen is cross-linked with an antibody reactive with CD45 antigen to enhance lymphocyte activation and function. The antibody heteroconjugates, methods and compositions of this invention are useful in the regulation of lymphocytes leading to improved cellular immune responses in the treatment of diseases such as infectious disease, cancer, AIDS and autoimmune disease.

BACKGROUND OF THE INVENTION

T lymphocytes, also referred to in the art as T cells, are known to mediate immune responses in at least two distinct ways. Cytotoxic T cells are involved in the lysis of specific target cells, while helper T cells assist in the proliferation and differentiation of B cells, leading to the production of antigen-specific antibodies (see, e.g., J.W. Kimball (ed.), Introduction To Immunology, 2nd Ed., Chapters 11-13, Macmillan Publishing Co. (1986)]. Either type Of T cell is activated to perform its function via the interaction of the T cell receptor (Ti) or its associated CD3 complex (also referred to as the CD3/Ti receptor complex) on the T cell surface with an antigen on an antigen-presenting cell [see, e.g., E.L. Reinherz et al., "Clonotypic Surface Structure On Human T Lymphocytes: Functional And Biochemical Analysis Of The Antigen Receptor Complex", Immunol. Rev., 81:95-129 (1984)].

It has been found, however, that T cells respond to an antigen only when that antigen in associated with a particular MHC (major histocompatibility complex)-encoded antigen on the antigen-presenting cell. T cell recognition of an antigen is said therefore to be restricted by the class of MHC-encoded product associated with the antigen. The result of this MHC restriction in that cytotoxic T cells are activated by antigens in association with class I MHC antigens and helper T cells are activated by antigens associated with class II MHC antigens [see, e.g., J.W. Kimball (ed.), Introduction To Immunology, supra at pp. 286-89 and pp. 348-58].

Furthermore, it has been recognized that the class II MHC-restricted T cells express, in addition to the CD3/Ti receptor complex, a cell surface antigen termed CD4, whereas the class I MHC-restricted T cells express a CD8 cell surface antigen. This association between MHC restriction and CD antigen expression has led to the hypothesis that, in T cell activation; the respective MHC antigens are the natural ligands for the CD antigens, i.e., the CD4 antigen on helper T cells interacts with the class II MHC molecules on the antigen-presenting cells (e.g., macrophage) and the CD8 antigen on cytotoxic T cells interacts with the class I MHC molecules on specific target cells (e.g., virus-infected cells) [see, e.g., F. Emmrich et al., "Cross-Linking Of The T Cell Receptor Complex With The Subset-Specific Differentiation Antigen Stimulates Interleukin 2 Receptor Expression In Human CD4 And CD8 T Cells", Eur. J. Immunol., 17:529-34 (1987)]. In addition, it has been proposed that the recognition of antigen by T cells may occur via a quaternary complex wherein the CD3/Ti receptor complex on the T cell surface, in association with a CD antigen such as CD4 or CD8, "sees" the antigen in association with the appropriate MHC-encoded antigen [see, e.g., P. Anderson et al., "Cross-Linking Of T3 (CD3) With T4 (CD4) Enhances The Proliferation Of Resting T Lymphocytes", J. Immunol., 139 (No. 3):678-82 (1987)].

As used in this application, CD antigens are naturally-occurring cell surface differentiation antigens defined by the reactivity of monoclonal antibodies (mAbs) on the surface of cells, as designated by an International Workshop whose function is to provide a unified nomenclature for these antigens [see, e.g., A.J. McMichael (ed.), Leukocyte Typing III, Oxford University Press (Oxford, U.K. 1987)]. A large number of CD antigens have been molecularly cloned and are therefore fully characterized [Id.]. Well known CD antigens include the pan-T antigens, CD3, CD2, CD5, CD6 and CD7, a CD antigen specific for the helper T cell subset, CD4, and a CD antigen specific for the suppressor T cell subset, CD8 [see, e.g., J. A. Ledbetter et al., in Perspectives In Immunogenetics And Histocompatibility, Vol. 6, E. Heise (ed.), Lymphocyte Surface Antigens 1984, pp. 325-40, American Society For Histocompatibility And Immunogenetics (New York 1984)]. CD28 is an antigen present on a majority of T cells [Yamada et al., Eur. J. Immunol. 15:1164-1169 (1985)].

Although these differentiation antigens are thought to function as receptors as described above and have been linked to signal transduction in T cells, their exact function in T cell activation is not known and is the subject of intensive research.

For example, studies have been carried out to determine the role of CD antigens in T cell activation on the molecular level. It is known that the reaction of the CD3/Ti receptor complex with either mAb or antigen leads to the generation of a transmembrane "signal" within the T cell. This signal is often characterized by the production of inositol phosphates [1, 2 and 3] and an increase in the concentration of cytoplasmic free calcium ($[Ca^{2+}]i$) [see, e.g., J. B. Imboden et al., "Transmembrane Signalling By The T Cell Antigen Receptor", J. Exp. Med., 161:446-56 (1985) and J.B. Imboden et al., "Antigen Recognition By A Human T Cell Clone Leads To Increases In Inositol Trisphosphate", J. Immunol., 138 (No. 5):1322-24 (1987)].

The generation of this signal may not be sufficient, however, to stimulate the T cell to proliferate [Id. at 873]. Under experimental conditions, it has been found that when the CD3 antigen on the T cell surface is cross-linked, e.g., by reacting the cell with a cross-linked antibody, the antibody having been cross-linked either in the presence of accessory cells (e.g., by the interaction of the monocyte Fc receptor with the Fc portion of an antibody to CD3) or by immobilization of the antibody on a solid support, T cell proliferation results.

Thus, mAbs to the CD3/Ti receptor complex that have been coupled to a solid support stimulate T cells to proliferate [see, e.g. P. Anderson et al., supra and C. Walker et al., "Activation Of T Cells By Cross-Linking An Anti-CD3 Antibody With A Second Anti-T Cell Antibody: Mechanism And Subset-Specific Activation", Eur. J. Immunol., 17:873-80 (1987)], in part due to the cross-linking of the CD3 antigen and in part due to the prevention of internalization of the CD3/Ti receptor complex, which internalization tends to inhibit T cell activation [see, e.g., J.A. Ledbetter et al., "Valency Of CD3 Binding And Internalization Of The CD3 Cell-Surface Complex Control T Cell Responses To Second Signals", J. Immunol., 136:3945-52 (1986)]. There are, however, substantial difficulties with the use of an anti-CD3 immobilized on a solid support to enhance T cell activation in vivo for therapeutic applications. Such a use would involve injection of the solid support, usually small beads, into a patient which would entail certain dangers to the health of the patient, such as blocked or clogged arteries.

Furthermore, it has been proposed that under physiological conditions, even this CD3/Ti stimulation via cross-linking of the CD3 antigen exerts only a minimal signal which may require enhancement by interactions on the T cell surface between CD3/Ti and other CD antigens [see P. Anderson et al., supra at p. 678].

In addition, there is uncertainty as to the exact effect of CD antigen interactions in T cell activation. For, example, analysis of spontaneous CD4 loss variants [see P. Marrack et al., "The Major Histocompatibility Complex Restricted Antigen Receptor On T Cells II. Role Of The L3T4 Product", J. Exp. Med., 158:1077-91 (1983)], as well as gene transfer experiments [see, e.g., D. Gay et al., "Functional Interaction Between Human T-Cell Protein CD4 And The Major Histocompatibility Complex HLA-DR Antigen", Nature, 328:626-29 (1987)], suggest that the expression of CD4 augments T cell responses to specific antigen and that CD4 plays a particularly important role when concentrations of antigens are suboptimal or when the avidity of the T cell for antigen/MHC-encoded antigen is low [see, A. Regnier-Bigouroux et al., "Accessory Molecules And T Cell Activation I. Antigen Receptor Avidity Differentially Influences T Cell Sensitivity To Inhibition By Monoclonal Antibodies To LFA-I And L3T4", Eur. J. Immunol., 16:1385-90 (1986) and S. Shaw et al., "Susceptibility Of Cytotoxic T Lymphocyte (CTL) Clones To Inhibition By Anti-T3 And Anti-T4 (But Not Anti-LFA-I) Monoclonal Antibodies Varies with The 'Avidity' Of CTL-Target Interaction", J. Immunol., 134 (No. 5):3019-26 (1985)]. Furthermore, when mAbs reactive with CD3 and CD4 were immobilized on the same solid support, proliferation of T cells exposed to the immobilized antibodies was enhanced. Similarly, when mAbs to CD3 and CD8 were coupled to a solid support, proliferation of T cells was enhanced over that observed upon exposure to antibody to CD3 alone [see, P. Anderson et al., supra.]

However, studies with soluble CD4 monoclonal antibody indicate that the soluble antibody inhibits T cell responses, suggesting that CD4 may transmit a negative signal that inhibits T cell activation [see, e.g., J.P. Bank et al., J. Exp. Med., 163:1294 (1985); J.P. Tite et al., "The Role Of L3T4 In T Cell Activation: L3T4 May Be Both An Ia-Binding Protein And A Receptor That Transduces A Negative Signal", J. Cell. Mol. Immunol., 2:179-90 (1986); and P.M. Rosoff et al., "The Role Of The L3T4 Molecule In Mitogen And Antigen-Activated Signal Transduction", Cell, 49:845-53 (1987)].

B lymphocytes, also known as B cells are the precursors of antibody-producing (plasma) cells. When B cells are stimulated by an antigen, requiring the cooperation of helper T cells and macrophages, the B cells proliferate and differentiate into plasma cells and memory B cells. CD antigens that have been identified on B cells include CD19, CD20, CDw40, CD45, CD45R and Bgp95 (an antigen that has not yet received a CD designation) [McMichael, Leukocyte Typing III, supra].

Another CD antigen of interest is the CD45 leukocyte common antigen (L-CA, also known as T200 or Ly-5). CD45 encompasses a family of major glycoproteins ranging from a molecular weight (Mr) of 180 to 220 kDa that is restricted to cells of hematopoietic lineages [Trowbridge et al., Proc. Nat'l. Acad. Sci. USA 72:157-161 (1975); (Komuro et al., Immunogenetics 1:452-456 (1975); (Schied et al., Immunogenetics 9:423-433 (1979); (Dalchau et al., Eur. J. Immunol. 10:737-744 (1980); and (Omary et al., J. Exp. Med. 152:842-852 (1980)]. Distinct isoforms of CD45 arise from alternative mRNA splicing. These isoforms are differentially expressed on subpopulations of T and B lymphocytes. Some monoclonal antibodies (mAbs) to human CD45 antigen recognize epitopes shared by all CD45 isoforms on Mr 220, 205, 190 and 180 kDa. [Cobold et al., Leukocyte Typing III, supra, Chap. 15, pp 788-803]. However, other mAbs recognize only the 220 kDa isoform of CD45, designated CD45R, that is selectively expressed on B lymphocytes and a subpopulation of T cells. [Cobold, supra; Dalchau et al., J. Exp. Med., 153:753-765 (1981); Morimoto et al., J. Immunol. 134:1508-1515 (1985); and Ledbetter et al., J. Immunol. 135:1819-1825 (1985)]. Another mAb, UCHL-I, selectively binds to the 180 kDa species, which is restricted to cortical thymocytes and a subset of activated or memory T cells. [Smith et al., Immunology 58:63-70 (1986)].

Recently, the primary structures of rat [Thomas et al., Cell, 41:83-93 (1985) and Barclay, et al., EMBO J. 6:1259-1264 (1987)], mouse (Saga, et al., Proc. Nat'l. Acad. Sci. USA 83:6940-6944 (1986); W.C. Raschke, Proc. Nat'l. Acad. Sci. USA 84:161-165 (1987); Thomas et al., Proc. Nat'l. Acad. Sci. USA 84:5360-5363 (1987) and Saga et al., Proc. Nat'l. Acad. Sci. USA 84:5364-5368 (1987)], and human [Ralph et al., EMBO J. 6:1251-1257 (1987) and Streuli et al., J. Exp. Med. 166:1548-1566 (1987)] CD45 (L-CA) have been deduced from cDNA nucleotide sequences. CD45 is an integral membrane protein with a large 705-707 amino acid cytoplasmic segment, a 22 amino acid transmembrane segment, and an extracellular domain ranging from 400 to 550 amino acids. The various isforms of CD45 that are generated by alternative mRNA splicing of primary transcripts of a single gene have different extracellular domains, but have the same transmembrane and cytoplasmic segments [Barclay, supra; Ralph, supra and Streuli, supra]. The cytoplasmic segment has two homologous, highly conserved domains of approximately 300 residues.

Studies attempting to define the function of CD45 have yielded conflicting results [Cobold, supra]. mAbs to the human or mouse antigen have been reported to inhibit T and B cell proliferation [Harp et al., J. Immunol. 133:10-15 (1984); Bernabeu, et al., Eur. J. Immunol. 17:1461-1466 (1987) and Mittler et al., J. Immunol. 138:3159-3166 (1987)], antibody-forming cell production [Yakura, J. Exp. Med. 157:1077-1088 (1983)], and natural killer (NK) cell and cytotoxic T cell activity [Seaman et al., J. Immunol. 127:982-986 (1981); W. Newman, Proc. Nat'l. Acad. Sci. USA 79:3858-3862 (1982); Nakayama et al., Proc. Nat'l. Acad. Sci. USA 76:1977-1981 (1979) and Lefrancois et al., Nature (London) 314:449-452 (1985)]. However, under certain conditions, anti-CD45 mAbs have also been reported to augment T cell proliferation [Cobold, supra; Ledbetter, supra and Martorell, et al., Eur. J. Immunol. 17:1447-1451 (1987)]. Thomas, supra, have suggested that the 80 kDa cytoplasmic portion of the molecule with its two conserved homologous domains may play a critical role in CD45 function. Recently, these domains were found to be homologous to a major low-molecular weight protein tyrosine phosphatase isolated from both the soluble and particulate fractions of human placenta [Tonks, et al., J. Biol. Chem., 263:6722-6730 (1988); and Charbonneau, et al., Proc. Nat'l. Acad. Sci. USA 85:7182-7186 (1988)]. This suggests that CD45 is a membrane-bound protein tyrosine phosphatase which may function by interacting with other membrane-associated molecules.

M.A. Lin et al disclose in Proc. Natl. Acad. Sci. USA (1985), 82 (24) 8648-52 heteroantibody duplexes formed by joining covalently an antibody to the T3 complex associated with the $\alpha,\beta$-receptors of human mature T-cells and a second antibody specific for surface antigen on a human B-lymphoma. The heteroconjugate induces the lysis of B-lymphoma cells by human T8[+] CTLs.

EP-A-0235805 discloses a cocktail of anti-T-cell monoclonal antibodies of different specificity, at least one of which being of the IgG class and at least one other being of the IgM class. While IgG type antibodies are used to effectively reach the lymphoid and transplanted tissue, the IgM type antibodies are used to inactivate circulating T-cells in the mammalian host.

## SUMMARY OF THE INVENTION

The present invention clarifies some of the uncertainties discussed above and provides antibody heteroconjugates comprised of at least two antibodies reactive with different surface antigens expressed on lymphocytes, such as T cells, the antibodies being cross-linked to each other. Certain of these heteroconjugates enhance signal transduction in the activation of T cells as determined by their ability to increase ($[Ca^{2+}]i$) in T cells at protein concentrations approximately two orders of magnitude lower than that seen with antibody to CD3 antigen alone.

A first embodiment of the invention relates to a heteroconjugate useful in the regulation of lymphocytes comprising at least two molecules selected from antibodies and ligand proteins capable of binding to antigens on lymphocytes, said molecules being cross-linked to each other, each molecule being reactive with a different antigen located on the surface of individual lymphocytes; preferably the heteroconjugate is an antibody heteroconjugate useful in the activation of T-lymphocytes comprising at least two antibody molecules cross-linked to each other, each antibody being reactive with a different antigen located on the surface of T-lymphocytes.

In addition, the heteroconjugates of the invention include those capable of inhibiting activation and function of lymphocytes.

According to a preferred embodiment of the invention, a monoclonal antibody reactive with the CD3/Ti receptor complex or its component parts is cross-linked to a monoclonal antibody, reactive with a second antigen on the T cell surface, such as the CD2, CD4, CD6 or CD8 antigen, to form heteroconjugates that enhance signal transduction in the activation of T cells. According to another preferred embodiment, a monoclonal antibody reactive with the CD5 T cell surface antigen is cross-linked to a monoclonal antibody reactive with a second antigen on the T cell surface, such as the CD4, CD6 or CD8 antigens. A particularly preferred embodiment relates to a heteroconjugate comprised of an antibody to CD3 cross-linked to an antibody to CD4.

According to yet another preferred embodiment, a monoclonal antibody reactive with the CD45 T cell surface antigen is cross-linked to a monoclonal antibody reactive with a second antigen on T cell surfaces such as the CD2, CD3, CD5 or CD28 antigen, to form heteroconjugates that inhibit activation and proliferation of lymphocytes. A particularly preferred embodiment relates to a heteroconjugate comprised of an antibody CD3 cross-linked to antibody reactive with CD45 antigen or the isoforms of CD45.

The present invention also encompasses in vitro methods of treating lymphocytes with the heteroconjugates of the invention to enhance or inhibit activation of lymphocytes in the production of cellular immune responses. The heteroconjugates of this invention may therefore be used in pharmaceutical compositions such as those comprising a pharmaceutically effective amount of at least one heteroconjugate of the invention and a pharmaceutically acceptable carrier. Thus, the heteroconjugates,methods and compositions of the invention can be used in immunotherapy to enhance or regulate immune responses, for example, in the treatment of infectious diseases, cancer, AIDS and autoimmune diseases.

## BRIEF DESCRIPTION OF THE DRAWINGS

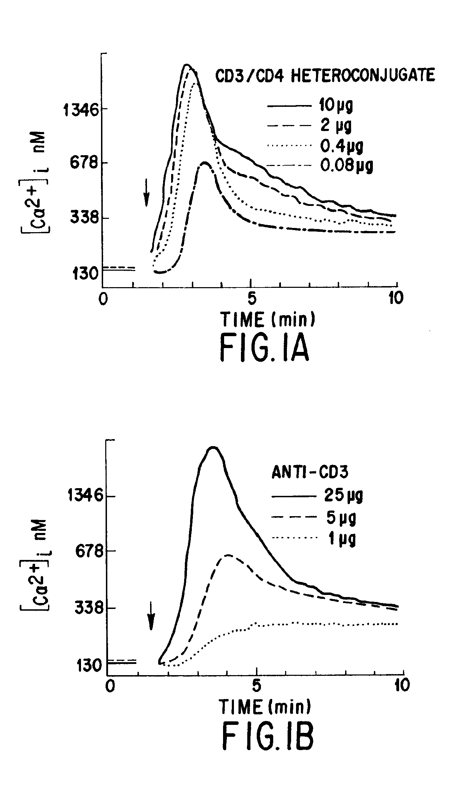

Figure 1 depicts a comparative graphical presentation of the increase in the concentration of cytoplasmic free calcium ($[Ca^{2+}]i$) over time upon stimulation of peripheral blood lymphocytes with varying concentrations of either (A) a CD3/CD4 heteroconjugate of one embodiment of the invention, G19-4/G17-2, or (B) the anti-CD3 monoclonal antibody, G19-4.

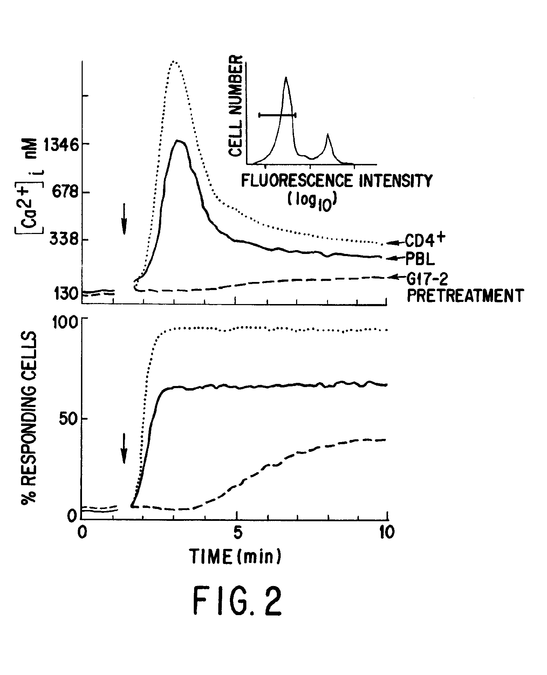

Figure 2 depicts comparative graphical presentations of the $[Ca^{2+}]i$ response and percentage of responding cells over time upon stimulation of either peripheral blood lymphocytes (PBL) or $CD4^+$ T cells with a CD3/CD4 heteroconjugate of the invention, G19-4/G17-2. The inhibitory effect of pretreatment of the cells with the anti-CD4 monoclonal antibody, G17-2, followed by stimulation with the heteroconjugate is also shown.

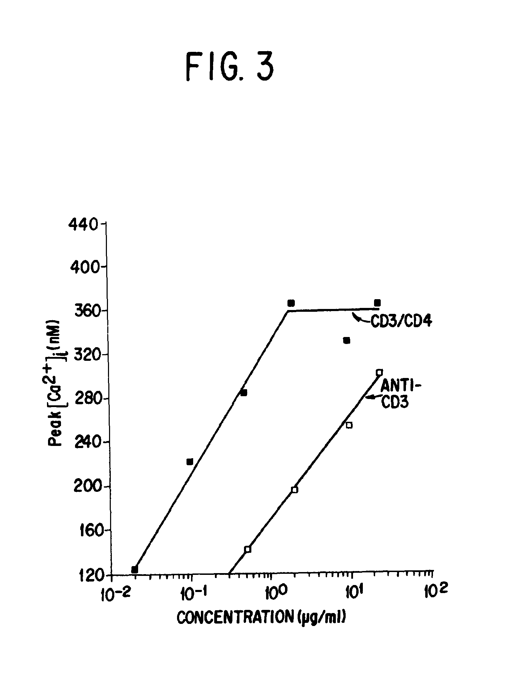

Figure 3 depicts comparative titrations of the $[Ca^{2+}]i$ activity of the G19-4/G17-2 heteroconjugate of the invention (■) or the anti-CD3 monoclonal antibody, G19-4 (□), on $CD4^+$ T cells in the presence of 5 mM EGTA.

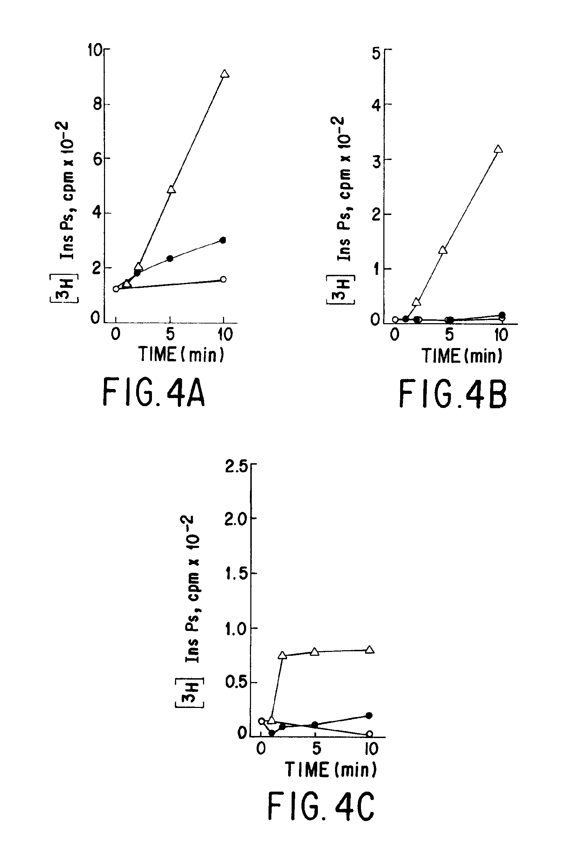

Figure 4 depicts graphical comparative presentations of the increase in inositol phosphates over time upon stimulation of $CD4^+$ T cells with anti-CD3, G19-4 (●) or a CD3/CD4 heteroconjugate of the invention, G19-4/G17-2 (△) at 1 μg/ml. The inositol phosphate levels in unstimulated controls (0) at t = 0 and t = 10 min are shown. Panel A depicts the increase in inositol phosphate 1 ($InsP_1$), panel B depicts the increase in inositol phosphate 2 ($InsP_2$) and panel C depicts the increase in inositol phosphate 3 ($InsP_3$).

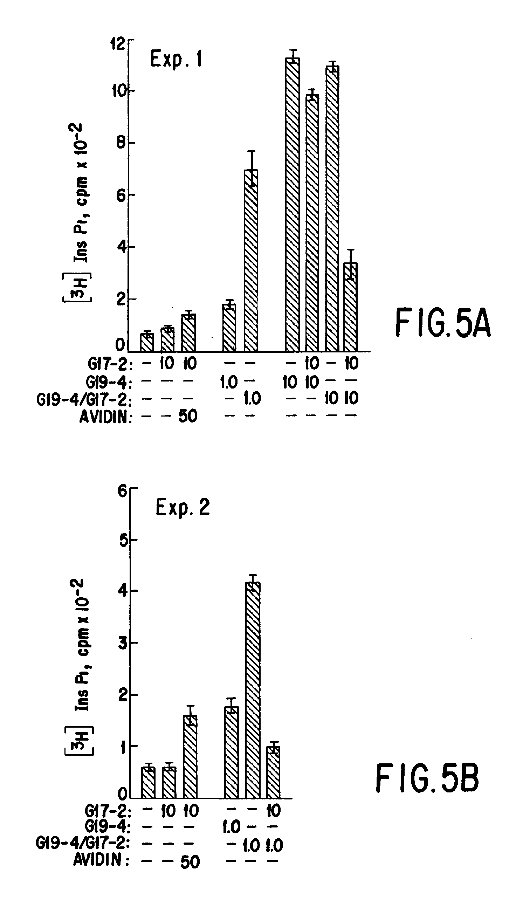

Figure 5 depicts two independent experiments wherein the increase in $InsP_1$ is measured following the stimulation of $CD4^+$ T cells, pretreated with biotin-conjugated anti-CD4 (G17-2), with either anti-CD3 (G19-4), G17-2, a CD3/CD4 heteroconjugate (G19-4/G17-2) or avidin and combinations thereof as indicated.

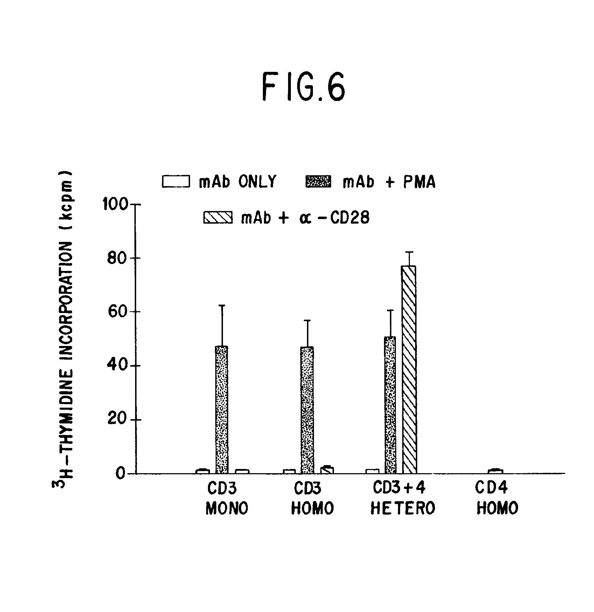

Figure 6 depicts the effects of an anti-CD3 monoclonal antibody (CD3 Mono), a CD3/CD3 homoconjugate (CD3 Homo), a CD4/CD4 homoconjugate (CD4 Homo) and a CD3/CD4 heteroconjugate of the invention (CD3 + 4 Hetero) on T cell proliferation as indicated by [$^3$H]-thymidine incorporation by $CD4^+$ T cells, with or without PMA (phorbol myristate acetate) or antibody to CD28.

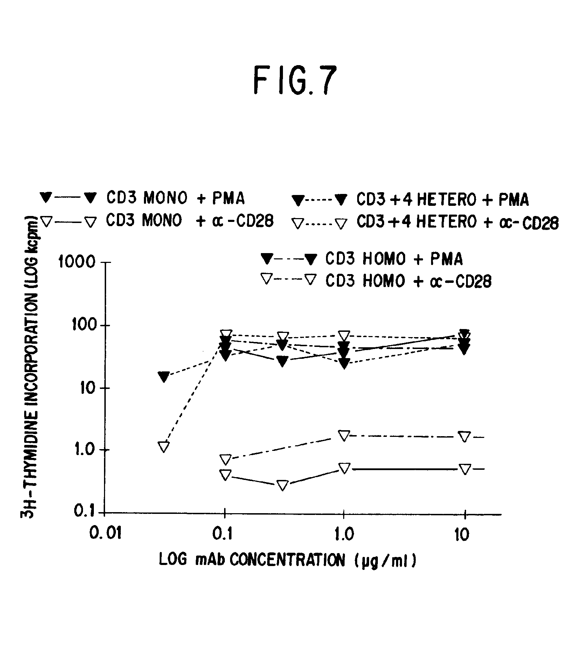

Figure 7 depicts a comparative graphical presentation of the proliferative response of $CD4^+$ T cells as indicated by $^3$H-thymidine incorporation vs. concentration of either G19-4 (CD3 Mono) + PMA

$$(\blacktriangledown\!\!-\!\!\blacktriangledown),$$

G19-4 + anti-CD28 (α-CD28) (▽—▽), a CD3/CD4 heteroconjugate (CD3 + 4 Hetero) + PMA

$$(\blacktriangledown\cdots\blacktriangledown),$$

a CD3/CD4 heteroconjugate + α-CD28 (▽—▽), a CD3/CD3 homoconjugate (CD3 Homo) + PMA

$$(\blacktriangledown\cdots\blacktriangledown)$$

or a CD3/CD3 homoconjugate + α-CD28 (▽—·—▽).

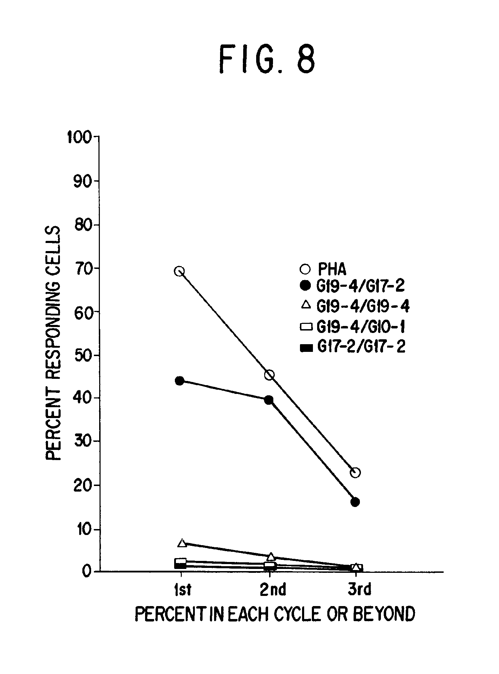

Figure 8 depicts the percentage of CD4[+] T cells past the first, second or third cell cycles after stimulation of the T cells for 3 days with either PHA (phytohemagglutinin), a CD3/CD4 heteroconjugate of the invention, G19-4/G17-2, a CD3/CD3 homoconjugate, G19-4/G19-4, a CD3/CD8 heteroconjugate as control, G19-4/G1O-1, and a CD4/CD4 homoconjugate, G17-2/G17-2.

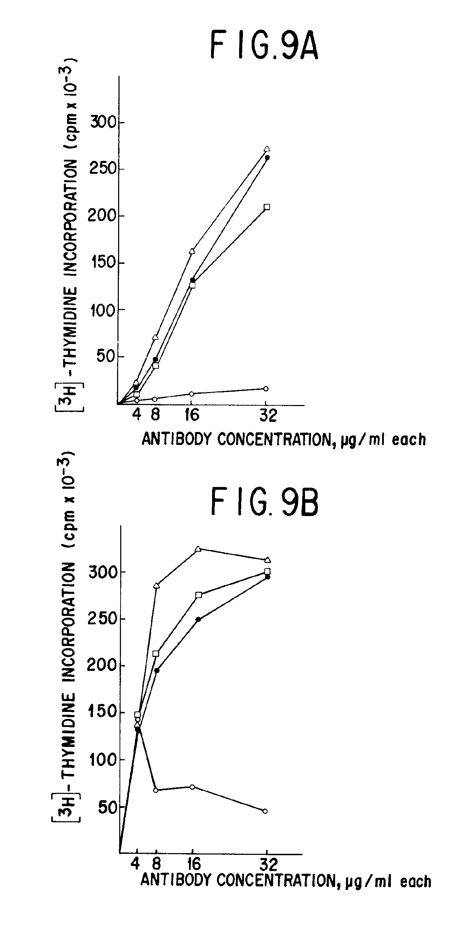

Figure 9 depicts a comparative graphical presentation of inhibition of the proliferative response of T cells induced by immobilized anti-CD3 mAb G19-4 as indicated by [$^3$H]-thymidine incorporation either alone (●) or vs. concentration of either anti-CD45 mAb 9.4 (□) or anti-p97 mAb 96.5 (□), without (A) or with (B) PMA, (proliferation with anti-CD45 mAb 9.4 in solution (△) is also shown for comparison; means are shown; standard errors were < 15%).

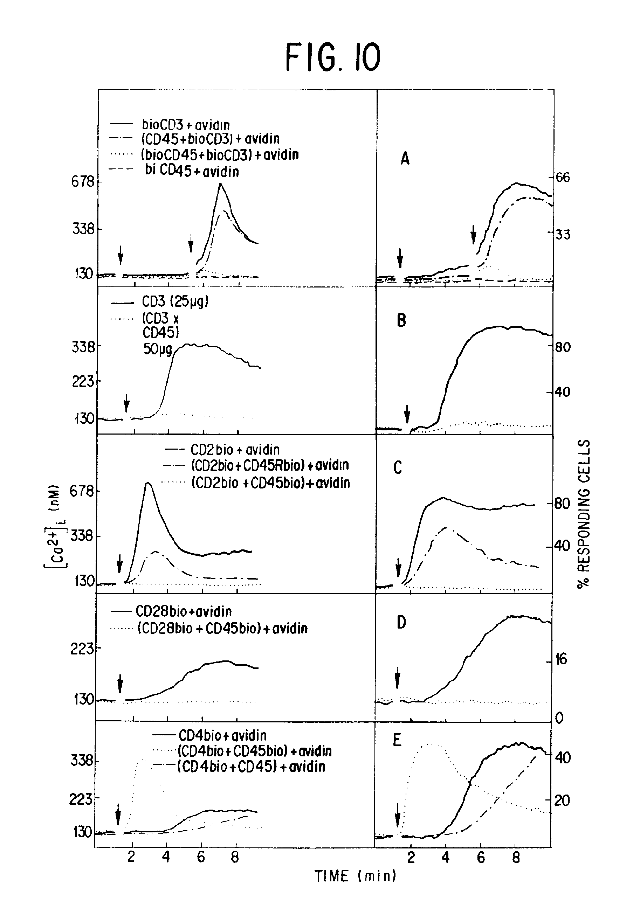

Figure 10 are graphs depicting the regulation of signal transduction in T cells by the CD45 receptor as determined by increases in [Ca$^{2+}$]i; over time after cross-linking receptors on T cells either alone or together with anti-CD45 mAb 9.4 or anti-CD45R mAb 3AC5 as described in Example 4, infra. (Mean [Ca$^{2+}$]i is shown on the left, and percentage of responding cells is shown on the right for each experiment; arrows indicate time of addition of mAbs or avidin).

DETAILED DESCRIPTION OF THE INVENTION

In order that the invention herein described may be more fully understood, the following detailed description is set forth.

The present invention relates to novel antibody heteroconjugates and their use in the enhancement or inhibition of lymphocyte activation and function. More particularly, the invention relates to heteroconjugates comprised of at least two antibodies cross-linked to each other, each antibody being reactive with a different lymphocyte cell surface antigen. Preferably, for enhancement of activity one of the antibodies of the conjugate is reactive with the T cell receptor (Ti) or its associated CD3 antigen complex and the other antibody is reactive with a second T cell surface antigen, such as the CD antigens (e.g., CD2, CD4, CD6, or CD8). Alternatively, one of the antibodies of the conjugate is reactive with the CD5 antigen and the other antibody is reactive with a second T cell surface antigen such as CD4, CD6 or CD8. The heteroconjugate CD4/CD45 also leads to enhanced T cell activity. For inhibition, one of the antibodies of the heteroconjugates is preferably reactive with the CD45 family of antigens (isoforms), and the, other antibody is reactive with a T cell surface antigen such as CD2, CD3, CD5 or CD28.

Without being bound by theory, it is believed that the heteroconjugates described herein, when reacted with lymphocytes, act by binding to their respective antigens on the surface of the cell, bringing those antigens into close proximity to each other, a step that may mimic the coclustering that occurs between the T cell receptor and CD4 upon contact of helper T cells with antigen-presenting cells during T cell activation [see, e.g., A. Kupfer et al., "Coclustering Of CD4 (L3T4) Molecule With The T-Cell Receptor Is Induced By Specific Direct Interaction Of Helper Interaction Of Helper T Cells And Antigen-Presenting Cells", Proc. Nat'l. Acad. Sci. USA. 84:5888-5892 (1987)]. This interaction between T cell antigens on the cell surface appears to enhance CD3/Ti-mediated transmembrane signalling and thus T cell activation.

While the mechanism of the CD45 antigen's effect on lymphocytes is not understood, the CD45 heteroconjugates described herein may function by modifying other lymphocyte receptors functionally when brought into close physical association with them, for example resulting in inhibition after aggregation of certain CD receptors, such as the CD3 antigen. The recently described homology between the cytoplasmic domains of CD45 and the major protein tyrosine phosphatase of human placenta suggest that the former is in fact a membrane-bound protein tyrosine phosphatase that functions to modify signal transduction in leukocytes by dephosphorylating key tyrosyl residues on other membrane-associated molecules such as on the zeta chain of CD3. [Klausner et al., J. Biol. Chem. 262:12654-12659 (1987)]. CD45 could also modify protein tyrosine kinase activity associated with CD4 that would normally function to regulate antigen-driven signal transduction. [Rudd et al., Proc. Nat'l. Acad. Sci. USA 85:5190-5194 (1988)]. Alternatively, the CD4-associated tyrosine kinase may phosphorylate CD45 antigen, on its tyrosyl residues and alter its activity. This could account for the unique enhancement activity obtained as, a result of cross-linking the CD4 and CD45 receptors as shown in the examples, infra. This model would predict that phosphorylation of key tyrosyl residues precedes and is necessary for the mobilization of calcium. An integral membrane form of the enzyme that functions only in close proximity with other membrane-associated proteins would serve to localize protein tyrosine kinase/phosphatase regulation to specific cell surface molecules. Thus, it is believed that the heteroconjugates of the invention can act to enhance or inhibit lymphocyte activation and function.

It should be noted that the T cell regulation, i.e. inhibition, according to this invention may be a pan-T cell regulation or the regulation of a particular subset or clone of T cells. Pan-T cell regulation results from contacting the T cells with heteroconjugates that regulate the total pool or population of T cells. These heteroconjugates are comprised of antibodies reactive with T cell antigens found on all T lymphocytes. One such heteroconjugate is the CD3/CD2 heteroconjugate of the invention, CD3 and CD2 being pan-T cell antigens [see, e.g., J. A. Ledbetter et al., in Perspectives In Immunogenetics And Histocompatibility, supra, Table 1].

However, by use of the appropriate heteroconjugate, a more specific regulation can be obtained. For example, in cases where it is desired to activate only a specific subpopulation of T cells, such as the helper or suppressor subset, this can be achieved by constructing a heteroconjugate wherein one of the antibodies is reactive with that particular T cell subpopulation, i.e., one antibody must be reactive with an antigen specific for that subpopulation. The other antibody of the conjugate is preferably specific for a pan-T cell antigen. According to this embodiment then, CD3/CD4 or CD5/CD4 heteroconjugates of the invention specifically enhance the activation of helper T cells (CD4 being specific for the helper T cell subset) while CD3/CD8 or CD5/CD8 heteroconjugate specifically enhances the activation of the suppressor T cell subpopulation (CD8 being specific for the suppressor T cell subset).

Alternatively, a CD3/CD45 heteroconjugate of the invention specifically inhibits the activation of T cells bearing CD45 antigens. Since isoforms of the CD45 receptor are also expressed on subsets of T cells, heteroconjugates of the invention that contain an antibody reactive with CD45 isoforms may also be used for regulation of those subsets of T cells.

Finally, where clonotypic regulation is desired, i.e., regulation of specific T cell clones, the heteroconjugate of the invention will contain an anti-clonotypic (Ti) antibody as one of its components, i.e., an antibody that reacts with the variable region of the T cell receptor of the clone sought to be regulated. Thus, in cases where a clonotypic T cell is unable to adequately see or respond to a particular antigen, e.g., a tumor or viral antigen, or is suppressed in its response, a heteroconjugate of this invention comprising an anti-clonotypic antibody specific for that T cell (Ti/CD4, Ti/CD8, Ti/CD6, Ti/CD2) may be used to regulate those T cell clones. Alternatively, for inhibition of a particular clone of T cells, a heteroconjugate composed of Ti/CD45 may be used (or Ti/CD45 isoform). According to this embodiment, the Ti component of the heteroconjugate can be viewed as essentially taking the place of the antigen in the regulation of the specific T cell. The other antibody of the heteroconjugate may be a pan-T cell-specific or subset-specific antibody or antibody reactive with CD45 or CD45 isoform.

Thus, the antibodies that comprise the heteroconjugates of the invention include any antibody reactive with a lymphocyte surface antigen. According to a preferred embodiment, one of the antibodies of the conjugate is reactive with the Ti or CD3 T cell antigen. According to a further preferred embodiment, an antibody reactive with the CD3 T cell surface antigen is cross-linked to an antibody reactive with the CD4 antigen on the T cell surface.

Other antibodies that may comprise the heteroconjugates of the invention include, but are not limited to, antibodies reactive with other CD antigens, e.g., CD2, CD5, CD6, CD7, CD8, CD28 and CD28 on lymphocytes [see, e.g., A. Bernard et al. (eds.), Leukocyte Typing, Springer Verlag (Heidelberg 1984); and J. A. Ledbetter et al., in Perspectives In Immunogenetics And Histocompatibility, supra]. Thus, according to another preferred embodiment, an antibody reactive with the CD5 T cell surface antigen is cross-linked to an antibody reactive with the CD4, CD6 or CD8 antigen to form a heteroconjugate of the invention. Yet another preferred embodiment is an antibody reactive with the CD3 T Cell surface antigen cross-linked to an antibody reactive with the CD45 antigen or isoform of CD45 on the lymphocyte cell surface.

The antibodies that comprise the heteroconjugates of the invention may be polyclonal or preferably, monoclonal, may be intact antibody molecules or fragments containing the active binding region of the antibody, e.g., Fab or F(ab')2; and can be produced using techniques well established in the art [see, e.g., publications describing the preparation of anti-CD3, anti-CD4, anti-CD5 and anti-CD2 monoclonal antibodies referenced in Examples 1-3 below]. In addition, monoclonal antibodies to many of the CD antigens such as the CD2, CD3, CD4 and CD5 antigens are commercially available (Becton Dickinson, Mountainview, CA) or may be obtained from the International Leukocyte Typing Workshops. If monoclonal antibodies are used, the antibodies may be of mouse or human origin or chimeric antibodies [see, e.g., V.T. Oi, "Chimeric Antibodies", BioTechniques. 4 (No. 3):214-221 (1986)]. Furthermore, the present invention also encompasses bispecific or recombinant monoclonal antibodies, wherein one specificity of the antibody is reactive with one T cell antigen and the other specificity is directed to a second T cell antigen. Such bispecific antibodies may be produced from a quadroma or trioma cell as described in United States Patent 4,474,893 or may be produced chemically [Brennan et al., Science:229:81-83 (1985)].

The antibodies that comprise the heteroconjugates of this invention can be covalently bound to each other by techniques known in the art such as the use of the heterobifunctional cross-linking reagents, GMBS (maleimidobutryloxy succinimide) or SPDP (N-succinimidyl 3-(2-pyridyldithio)propionate) [see, e.g., R.R. Hardy, "purification And Coupling Of Fluorescent Proteins For Use In Flow Cytometry", Handbook Of Experimental Immunology, Volume 1, Immunochemistry, D.M. Weir et al. (eds.), pp. 31.4-31.12 4th Ed., (1986) for a discussion of conventional antibody coupling techniques].

It is to be understood, therefore, that the present invention encompasses any heteroconjugate comprised of at least two molecules reactive with different lymphocyte antigens on the same lymphocyte, the heteroconjugate acting to enhance or inhibit the activation and function of the lymphocyte. The invention contemplates heteroconjugates reactive with antigens on B cells as well as T cells.

It is apparent that in addition to antibodies, the heteroconjugates of the present invention may incorporate ligands capable of binding to lymphocyte receptors. For example, CD4 receptor has been shown to be reactive with the ligand gp120 (a glycoprotein receptor of the Human Immunodeficiency Virus or "HIV") [Linsley et al., J. Virology 62:3695-3702 (1988)]. Thus, a heteroconjugate consisting of anti-CD3 antibody cross-linked to the ligand gp120 would be used to enhance T cell activity by cross-linking the CD3 and CD4 receptors. Other ligand conjugates consisting of a ligand reactive with a T cell receptor cross-linked to a ligand reactive with a different T cell receptor are also contemplated.

The heteroconjugates of the invention and their use in T cell activation are exemplified by a preferred embodiment in which an antibody reactive with the CD3 antigen on the surface of T cells was conjugated to an antibody reactive with the CD4 antigen to form a novel CD3/CD4 heteroconjugate of the invention. Treatment of CD4$^+$ T cells with this heteroconjugate resulted in enhanced signal transduction in the helper subset of T cells as indicated by a significant increase in intracellular $[Ca^{2+}]i$ mobilization and substantial increases in inositol phosphate 1, 2 and 3 formation as compared with treatment of the cells with antibody to CD3 alone. This signal-enhancing activity of the CD3/CD4 heteroconjugate was not simply due to oligomerization, since CD3/CD3 and CD4/CD4 homoconjugates or mixtures thereof did not show this increased activity. And, it has been previously demonstrated in the art that mixtures of anti-CD3 and anti-CD4 antibodies decrease rather than enhance this activity [see, e.g., P.M. Rosoff et al., supra]. Furthermore, the enhanced activity demonstrated by the heteroconjugate was inhibited by pretreatment of the T cells with soluble monoclonal antibody to CD4.

In addition, the CD3/CD4 heteroconjugate exhibited a novel functional activity in T cell activation, i.e., the heteroconjugate induced the proliferation of CD4+ T cells in the presence of a monoclonal antibody reactive with CD28 and was fully comitogenic with CD28 stimulation even at concentrations as low as 100 ng/ml. Such comitogenicity was not found using antibody to CD3 alone or CD3/CD3 or CD4/CD4 homoconjugates. Furthermore, it was found that the CD3/CD4 heteroconjugate of the invention, in combination with CD28 monoclonal antibody, was able to significantly drive the T cells through the cell cycle, with a significant fraction of the cells entering a second cycle within the first three days of stimulation. In contrast, antibody to CD3 alone or CD3/CD3 or CD4/CD4 homoconjugates did not induce significant cell cycle transition.

Additionally, it was found that the CD3/CD4 heteroconjugate caused the modulation of both the CD3 and CD4 antigens on the T cell surface, leading to a decrease in the surface expression of CD4. This result indicates that the heteroconjugate is not simply anchoring the T cell receptor to the T cell surface as was previously shown to be the case using immobilized antibody to CD3 [see, e.g., J.A. Ledbetter et al., J. Immunol., supra].

The results obtained with the CD3/CD4 heteroconjugate demonstrate that the CD4 antigen on the T cell surface plays an active positive role in signal transduction during T Cell activation of CD4+ helper T cells via a physical interaction between the CD3/Ti receptor complex and the CD4 antigen.

The molecular mechanisms that underlie the CD3/CD4 heteroconjugate's ability to augment signal transduction are not well understood. At least one effect of the CD4 interaction with CD3/Ti appears to be the enhancement of inositol phosphate production, suggesting that CD4 may promote CD3/Ti-induced hydrolysis of membrane phosphoinositides, leading to the production of inositol trisphosphate. This is supported by our experimental data indicating that the inositol phosphate-generating activity of the CD3/CD4 heteroconjugate is inhibited by reaction of the cells with soluble CD4 monoclonal antibody. This inhibition may be a consequence of antibody-induced dissociation of CD4 and CD3/Ti, if CD3/Ti alone is a less efficient signal transducer. Alternatively, independent interaction of CD4 with its antibody may transmit a negative signal that inhibits the generation of inositol phosphates.

Since the generation of inositol trisphosphate ($InsP_3$) is believed to mediate mobilization of cytoplasmic calcium in T cells, the interaction of CD4 with CD3/Ti also appears to promote release of calcium from intracellular stores. Comparison of the dose-response curves of the CD3/CD4 heteroconjugate vs. antibody to CD3 in $[Ca^{2+}]$ mobilization indicates that a detectable $[Ca^{2+}]$; response is achieved from the interaction of far fewer receptors on the T cell when CD4 and CD3/Ti are stimulated in proximity, i.e., by use of the CD3/CD4 heteroconjugate, than when CD3/Ti is stimulated alone (see Figure 3). Thus, one property of the CD3/CD4 heteroconjugate of the invention is its ability to activate T cells at low levels of receptor occupancy.

Because it is known that inositol phosphate generation and calcium mobilization are signals associated with the activation of T cells, leading to T cell proliferation, the data presented herein clearly demonstrates that, regardless of the mechanism of action, the CD3/CD4 heteroconjugate of the invention is, useful in the enhancement of T cell activation and proliferation.

The present invention also encompasses other heteroconjugates that, like the CD3/CD4 heteroconjugate, display enhanced signalling capabilities in T cell activation. For example, a CD3/CD2 heteroconjugate, a CD3/CD6 heteroconjugate, a CD3/CD7 heteroconjugate and a CD3/CD8 heteroconjugate of the invention also displayed enhanced activity in the mobilization of calcium. Furthermore, according to this invention, several anti-CD5-containing heteroconjugates were also constructed (e.g., a CD5/CD4 heteroconjugate, a CD5/CD6 heteroconjugate and a CD5/CD8 heteroconjugate) and displayed enhanced activity in the mobilization of $[Ca^{2+}]$ in T cells.

The heteroconjugates of the invention incorporating an antibody reactive with CD45 antigen or its isoforms and useful for inhibition of lymphocyte activation and function, or enhancement of T cell activation and function, are exemplified by a preferred embodiment in which an antibody reactive with the CD3 antigen was conjugated to an antibody reactive with the CD45 antigen to form a novel CD3/CD45 heteroconjugate of the invention. Treatment of T cells with this heteroconjugate resulted in abrogation of CD3-mediated activation (transmembrane signal) of the T cells as indicated by a significant decrease in intracellular calcium mobilization.

In addition, induction of interaction between various CD receptors on the surface of T cells such as CD3, CD2, CD5 or CD28, and the CD45 receptor was accomplished using monoclonal antibodies to cross-link the CD45 receptor on the lymphocyte with another CD receptor on the same lymphocyte. This cross-linking resulted in abrogation of the increase in the cytoplasmic free calcium concentration ($[Ca^{2+}]i$) that occurred from formation of homoaggregates of the T cell antigens induced by cross-linking common receptors on the cell surface. T cell proliferation initiated by immobilized anti-CD3 monoclonal antibody stimulation was inhibited by anti-CD45 antibody or anti-CD45R antibody when immobilized on the same surface, but not when in solution. Similarly, proliferation of T cells after stimulation of the CD2 and CD28 receptors by anti-CD2 and anti-CD28 antibodies, respectively, was inhibited when a CD45 antibody was cross-linked to either anti-CD2 antibody or anti-CD28 antibody, but not when an anti-CD45 antibody was bound to the cell surface separately. These results suggest that heteroconjugates consisting of antibodies reactive with CD45 or isoforms of CD45 such as CD45R and antibodies reactive with CD antigens on T cells may be useful to regulate the cells.

In contrast, the increase in $[Ca^{2+}]i$ induced by formation of homoaggregates of CD4 receptors on T cells was strongly amplified when the CD4 receptor was cross-linked to the CD45 receptor using monoclonal antibodies. Thus, a novel heteroconjugate consisting of antibody against CD4 and antibody reactive with CD45 or isoforms of CD45R on T cells may also be used to enhance T cell function of all CD4 positive cells or subpopulations of CD4 cells defined by expression of CD45 isoforms.

The heteroconjugates of the invention are useful to regulate lymphocytes and therefore, may be used to regulate cellular immune responses in disease states such as infectious diseases, cancer, AIDS, and autoimmune disorders. These heteroconjugates may be especially useful for the regulation of cellular immune responses in disease states where there is a defect or disregulation of T cells.

For example, it has been shown that CD45 isoforms are abnormally expressed in certain autoimmune diseases [Rose et al., Proc. Nat'l. Acad. Sci. (USA) 82:7389-7393 (1985)]. Therefore, heteroconjugates of the invention containing at least one molecule reactive with CD45 receptor or CD45 isoforms may be useful in regulating lymphocyte function in autoimmune diseases.

In addition, the heteroconjugates of the present invention may be useful in enhancing immune responsiveness in patients with Acquired Immunodeficiency Syndrome (AIDS). It is known that the CD4 antigen serves as the receptor for the Human Immunodeficiency Virus (HIV) that is responsible for AIDS. It is believed that CD4 is the receptor to which the gp120 envelope glycoprotein of HIV binds during infection of T cells with the virus [see, e.g., L.A. Lasky et al., Cell, 50:975 (1987) and M. Kowalski et al., Science, 237:1351 (1987)]. After HIV infection, the cell surface expression of CD4 is down regulated and levels of CD4 mRNA are decreased [see J.A. Hoxie et al., "Alterations In T4 (CD4) Protein And mRNA Synthesis In Cells Infected With HIV", Science, 234:1123-1127 (1986)]. It is also well established that AIDS patients display reduced immune responsiveness to soluble antigens [see, e.g., H.C. Lane et al., "Qualitative Analysis Of Immune Function In Patients With The Acquired Immunodeficiency Syndrome", New Eng. J. Med., 313 (No. 2):79-84 (1985)]. In vitro studies have shown that purified HIV gp120 can inhibit T cell responses to mitogenic stimulation, a result similar to the effect we have seen using soluble monoclonal antibody to CD4 [see D.L. Mann et al., J. Immunol., "HTLV-III Large Envelope Protein (gp120) Suppresses PHA-Induced Lymphocyte Blastogenesis", 138:2640-2644 (1987)].

Thus, it may be proposed that the immunosuppression seen in AIDS patients may be related to the effect of HIV infection on CD4 antigen expression, i.e., the down regulation of CD4 on the T cell that occurs after HIV infection may affect immune responsiveness by diminishing the signalling capability of the cell, leading to decreased T cell activation in response to antigen. In support of this model, we have found that CD3-stimulated increases in free calcium concentration are impaired in HIV-infected cells [G.P. Linette et al., "HIV-1 Infected T Cells Exhibit A Selective Transmembrane Signalling Defect Through The CD3/Antigen Receptor Pathway", Science, 241:573-576 (1988)]. These observations suggest that the heteroconjugates of the invention, particularly a CD3/CD4 heteroconjugate, may be useful for studying or treating T cell dysfunctions resulting from HIV infection.

The present invention also encompasses methods of treating lymphocytes, either in vitro or in vivo, with the heteroconjugates disclosed herein for the regulation of cellular immune responses in disease states.

According to one embodiment of the invention, the heteroconjugates con be utilized in the in vitro activation of T cells. This activation can be carried out by contacting T lymphocytes taken from a patient with at least one of the heteroconjugates of this invention in vitro whereby the T cells become activated and can then be reinfused into the autologous donor [see, e.g., S.A. Rosenberg et al., "Immunotherapy Of Cancer By Systemic Administration Of Lymphoid Cells Plus Interleukin 2", J. Biol. Resp. Modif., 3:5501-5511 (1984)]. This method of treatment may also involve the in vitro coincubation or preincubation of the T cells with other immunomodulators.

According to an alternative embodiment, the heteroconjugates may be used to activate T cells in vivo by injecting the heteroconjugates into a patient, possibly in conjunction with other agents such as interleukin-2 (IL-2), growth factors or agonistic antibodies such as CD5 [see, e.g., E.A. Clark et al., "Amplification Of The Immune Response By Agonistic Antibodies", Immunology Today, 7:267-270 (1986)] and CD28 (see Example 1 below). Regardless of the method of treatment, it may be useful to use heteroconjugates comprising antibody fragments such as Fab or F(ab')$_2$, chimeric antibodies or bispecific antibodies.

The heteroconjugates of the invention can also be administered in vivo using conventional modes of administration which include, but are not limited to, intravenous, oral, subcutaneous, intraperitoneal or intralymphatic. Intravenous administration is preferred.

The pharmaceutical compositions of the invention --comprising the heteroconjugates -- may be in a variety of dosage forms which include, but are not limited to, solid, semi-solid and liquid dosage forms such as tablets, pills, powders, liquid solutions or suspensions, suppositories, polymeric microcapsules or microvesicles, liposomes, and injectable or infusible solutions. The preferred form depends upon the mode of administration and the therapeutic application.

The heteroconjugate compositions may include conventional pharmaceutically acceptable carriers known in the art such as serum proteins such as human serum albumin, buffer substances such as phosphates, water or salts or electrolytes.

The most effective mode of administration and dosage regimen for the heteroconjugate compositions of this invention depends upon the severity and course of the disease, the patient's health and response to treatment and the judgment of the treating physician. Accordingly, the dosages of the heteroconjugates should be titrated to the individual patient. Nevertheless, an effective dose of heteroconjugate of this invention may be in the range of from about 1 to about 100 mg/m$^2$. For in vitro treatment of T cells, a dose of from about 200 μg - 2 mg of heteroconjugate/$10^9$ cells may be used.

In addition, the heteroconjugates of the invention may be used diagnostically to measure the receptor function of a given T cell surface antigen, e.g., CD antigen, in patients having diseases involving a defect or disregulation of T cells. Using a calcium assay as described herein for the measurement of signal transduction (see Example 1) and appropriate controls, one can measure a defect in CD function by comparing the activation, by a specific heteroconjugate of the invention, of T cells from a normal vs. a diseased donor. For example, the CD3/CD4 heteroconjugate of this invention enhances $[Ca^{2+}]$; mobilization at protein concentrations below that seen with soluble anti-CD3 alone. Thus, at these low doses, one is actually measuring the function of the CD4 receptor. This is reflected by the ability of soluble anti-CD4 to block the activity of the CD3/CD4 heteroconjugate at these doses (see Figure 2). Similarly, with the CD3/CD8 heteroconjugate at low doses, one is able to measure the function of the CD8 receptor. At these doses then, the heteroconjugate can be used to detect defects in a particular CD receptor function in various disease states, such as cancer and autoimmune disease. Alternatively, the calcium assays can be run at any dose with any heteroconjugate of the invention as long as the appropriate single antibody controls are carried out, i.e., the activity of the heteroconjugate must be compared to the activity of the unconjugated antibodies that make up the components of that heteroconjugate [see, e.g., European Patent Application 221,768 of E.L. Reinherz et al., published on May 13, 1987].

In order that the invention described herein may be more fully understood, the following examples are set forth. It should be understood that the examples are for illustrative purposes only and are not to be construed as limiting the scope of this invention in any manner.

EXAMPLE 1

The following example describes the preparation and characterization of a novel heteroconjugate of the invention. According to this embodiment, an antibody reactive with CD3 was cross-linked to an antibody reactive with CD4 to form a novel CD3/CD4 heteroconjugate that causes enhanced transmembrane signalling in the activation of CD4$^+$ (i.e., helper) T cells treated with the heteroconjugate.

Preparation of A CD3/CD4 Heteroconjugate Of The Invention

The CD3 and CD4 antibodies utilized to form the heteroconjugate of this embodiment were G19-4 (Balb/c, IgG$_1$) and G17-2 (Balb/c, IgG$_1$), respectively. These antibodies were prepared as described by J.A. Ledbetter and E. Clark, "Surface phenotype And Function Of Tonsillar Germinal Center And Mantle Zone B Cell Subsets", Human Immunology, 15:30-43 (1986) and J.A. Ledbetter et al., "An Immunoglobulin Light Chain Dimer With CD4 Antigen Specificity", Molecular Immunology, 24 (No. 12):1255-1261 (1987). In addition, other anti-CD3 and anti-CD4 monoclonal antibodies that can be used to construct the heteroconjugate of this embodiment are commercially available (e.g., T3, Coulter Co., FL and Leu-3, Becton Dickinson, CA, respectively). The G19-4 and G17-2 antibodies were purified from ascites fluid by salt precipitation and DEAE chromatography, followed by dialysis and filtration through 0.45 μ filters prior to use.

The two monoclonal antibodies were conjugated at a 1:1 molar ratio with the heterobifunctional cross-linking reagent, GMBS (Calbiochem, La Jolla, CA) and 5-iminothiolane HCl (Pierce Chemical Co., Rockford, IL) as described for phycoerythrin coupling by R.R. Hardy, supra. The heteroconjugate that resulted was separated from free antibody by Superose 6® (Pharmacia, Uppsala, Sweden) FPLC size exclusion chromatography and tested for reactivity with both CD3 and CD4 by testing the ability of the heteroconjugate to block the binding of directly fluorescein-conjugated anti-CD3 and anti-CD4 monoclonal antibodies. This CD3/CD4 heteroconjugate was designated G19-4/G17-2.

Enhanced Calcium Mobilization By The CD3/CD4 Heteroconjugate Of The Invention

The G19-4/G17-2 heteroconjugate was then tested for its ability to enhance the mobilization of cytoplasmic calcium in cells, compared to the activity of G19-4 (anti-CD3) alone. The cells used were human peripheral blood lymphocytes purified from the peripheral blood by centrifugation on Ficoll-Hypaque®, followed by adherence to plastic to remove the majority of monocytes.

Increases in $[Ca^{2+}]i$ within the cells were measured using indo-I (Molecular Probes, Eugene, OR) and a model 50 HH/2150 flow cytometer (Ortho Diagnostic Systems, Inc., Westwood, MA) as described previously by P.S. Rabinovitch et al., "Heterogeneity Among T Cells In Intracellular Free Calcium Responses After Mitogen Stimulation With PHA or Anti-CD3. Simultaneous Use Of Indo-I And Immunofluorescence With Flow Cytometry", J. Immunol., 137(No. 3): 952-961 (1986), incorporated by reference herein. Briefly, cells ($5 \times 10^7$/ml) were loaded with indo-I by incubation with its acetoxy-methyl ester (Molecular Probes, Junction City, OR) which is permeable through the cell membrane and is hydrolyzed in the cytoplasm to the impermeant trapped form. The incubation was carried out for 45 min at 37°C in medium containing the indicated indo-I concentration. Cells were then washed and resuspended in fresh medium at $2.5 \times 10^6$/ml and stored in the dark at room temperature until analysis. For each assay, indo-I-loaded cells were diluted to $1 \times 10^6$/ml with medium and equilibrated at 37°C. Measurements were performed using spectrofluorimetry and flow cytometry as described by Rabinovitch, supra. The histograms were analyzed by programs that calculated the mean indo-I violet/blue fluorescence ratio vs. time. In addition, the percentage of responding cells vs. time was analyzed by programs that first determined the value of the indo-I ratio that was two standard deviations about the ratio for control cells and then plotted the percentage of cells above this threshold value vs. time. There are 100 data points on the X (time) axis on all flow cytometric data.

As shown in Figure 1, the concentration of G19-4/G17-2 required to increase the concentration of $Ca^{2+}$ ($[Ca^{2+}]i$) was 1.5 - 2 orders of magnitude lower than the concentration required using G19-4 alone. Furthermore, the activity of the G19-4/G17-2 heteroconjugate was restricted to $CD4^+$ T cells, since enrichment for $CD4^+$ cells by immunofluorescence resulted in a corresponding increase in $[Ca^{2+}]i$ activity and percent of responding cells. Thus, as Figure 2 demonstrates, when peripheral blood lymphocytes were stimulated by the G19-4/G17-2 heteroconjugate, the mean maximal $[Ca^{2+}]i$ response for the cells was approximately 1300 nM and approximately 60% of the cells responded. However, when only the $CD4^+$ subset of cells was analyzed, the mean maximal $[Ca^{2+}]i$ response was >10,000 nM and >95% of the cells responded. Furthermore, the activity of the heteroconjugate on $CD4^+$ cells was almost completely inhibited by pretreatment of the cells at -5 min with 5 μg/ml G17-2 (anti-CD4) followed by 0.4 μg/ml of the heteroconjugate.

The $CD4^+$ cells were analyzed by gating on CD8 negative, CD11 negative, CD16 negative and CD20 negative cells as shown in the panel insert of Figure 2. This negative selection for $CD4^+$ cells was carried out by staining peripheral blood lymphocytes with phycoerythrin (PE)-conjugated 2H7, a monoclonal antibody reactive with CD20 (see, e.g. E.A. Clark et al., "Role Of The Bp35 Cell Surface Polypeptide In Human B-Cell Activation", Proc. Nat'l. Acad. Sci. USA, 82:1766-1770 (1985)], FC-2, a monoclonal antibody to CD16 (see, e.g., C. Anasetti et al., "Induction Of Calcium Flux And Enhancement Of Cytolytic Activity In Natural Killer Cells By Cross-Linking Of The Sheep Erythrocyte Binding Protein (CD2) And The Fc-Receptor (CD16)", J. Immunol., 139 (No. 6:)1772-1779 (1987)], G1O-1, a monoclonal antibody to CD8 [see, e.g., J.A. Ledbetter et al., "Covalent Association Between Human Thymus Leukemia-Like Antigens And CD8 Molecules", J. Immunol., 134:4250-4254 (1985)], and 60.1, a monoclonal antibody reactive with CD11 [see, e.g., J.P. Bunyon et al., "Differential Participation Of Epitopes On Alpha And Beta Chains Of The LFA Family In Neutrophil Aggregation As Defined By Workshop Monoclonal Antibodies", in Leukocyte Typing III, supra, pp. 844-47], and gating on the unstained cells, which were >95% CD4 positive.

Comparative titrations of G19-4 vs. the G19-4/G17-2 heteroconjugate were also carried out in the presence of 5 mM EGTA [ethylene glycol-bis (β-aminoethyl ether)-N,N, N',N'-tetraacetic acid], which chelates and thus removes extracellular calcium, to see if it was calcium mobilization from cytoplasmic sources that was being affected by the heteroconjugate. Figure 3 shows that there was approximately a two log increase in ability to mobilize calcium with the G19-4/G17-2 heteroconjugate in the presence of EGTA. In the presence of extracellular calcium, titrations to the end point indicated an approximately two-log increase in activity as well (data not shown).

In order to exclude that valency of binding to the CD3 or CD4 antigens was responsible for the effects of the CD3/CD4 heteroconjugate on $[Ca^{2+}]i$, we constructed CD3/CD3 and CD4/CD4 homoconjugates to compare with the CD3/CD4 heteroconjugate. The homoconjugates were constructed as described above for the CD3/CD4 heteroconjugate and have been designated G19-4/G19-4 and G17-2/G17-2, respectively. As shown in Table 1 below, neither the CD3/CD3 homoconjugate nor the mixture of CD3/CD3 plus CD4/CD4 homoconjugates mimicked the enhanced effects of the CD3/CD4 heteroconjugate on $[Ca^{2+}]i$. This data suggests that the enhanced activity of the CD3/CD4 heteroconjugate is not a function of the valency of the heteroconjugate and therefore presumably results from the physical proximity of the CD3 and CD4 molecules that occurs via interaction of those antigens with the heteroconjugate. Furthermore, heteroconjugates and homoconjugates were slightly less active than free antibodies in binding assays using indirect staining and flow cytometry (data not shown). Thus, the unique activity of the CD3/CD4 heteroconjugate is unlikely to be related to an effect on CD3 binding affinity contributed by the second specificity of the heteroconjugate.

Table I

| CD4[+] T Cell [Ca$^{2+}$]i Responses to CD3/CD4 Heteroconjugate Versus CD3/CD3 and CD4/CD4 Homoconjugates* | | | |
|---|---|---|---|
| Stimulation | Concentration μg/ml | Peak Mean [Ca$^{2+}$]i (nM) | Increase Above Basal |
| None | -- | 130 | 1.0 |
| CD3/CD4 Heteroconjugate | 10 | 15,600 | 120 |
| | 2 | 10,900 | 83 |
| | 0.4 | 4,140 | 32 |
| | 0.08 | 810 | 6.2 |
| CD3/CD3 Homoconjugate | 50 | 2,360 | 18 |
| | 10 | 559 | 4.3 |
| | 2 | 301 | 2.3 |
| CD4/CD4 Homoconjugate | 100 | 130 | 1.0 |
| | 50 | 130 | 1.0 |
| CD3/CD3 Homoconjugate + CD4/CD4 Homoconjugate | 25+25 | 590 | 4.5 |
| | 5+5 | 272 | 2.1 |
| | 1+1 | 251 | 1.93 |

Assays of [Ca$^{2+}$]i responses utilized peripheral blood lymphocytes loaded with indo-l and stained with PE-conjugated anti-CD20, anti-CD8 and anti-CD16 as described above. The unstained cells, comprising >95% CD4[+] cells, were analyzed. The peak mean response occurred within the first five minutes after stimulation.

Effects On Inositol Phosphate Synthesis By The CD3/CD4 Heteroconjugate Of The Invention

Because the mobilization of cytoplasmic calcium in T cells is thought to be mediated by the generation of inositol trisphosphate (InsP$_3$), we measured the increase in inositol phosphates after stimulation of purified CD4[+] T cells with either monoclonal antibody to CD3 alone or with the CD3/CD4 heteroconjugate.

The CD4[+] T cells were purified as described by C.H. June et al., "T-Cell Proliferation Involving The CD28 Pathway Is Associated with Cyclosporine-Resistant Interleukin 2 Gene Expression", Mol. Cell. Biol., 7 (No. 12): 4472-4481 (1987). Briefly, peripheral blood lymphocytes were obtained by leukophoresis of laboratory personnel, followed by Ficoll/Hypaque density gradient centrifugation. The CD4[+] subset of T cells were then isolated by negative selection with magnetic bead immunoabsorption after first coating the CD8[+], CD11[+], CD16[+], CD14[+], and CD20[+] cells with saturating amounts of the appropriate monoclonal antibody (the majority of antibodies having been referenced earlier, except for the anti-CD14 monoclonal antibody, 20.3, prepared as described by M. Kamoun et al., "Human Monocyte-Histiocyte Differentiation Antigens Identified By Monoclonal Antibodies", Clin. Immunology and Immunopathology, 29: 181-195 (1983)). This strategy took advantage of the reciprocal and nonoverlapping distribution of the CD4 and CD8 surface antigens on resting peripheral blood T lymphocytes. The cells were washed three times to remove unbound antibody, and then incubated with goat anti-mouse immunoglobulin-coated magnetic particles (Advanced Magnetics Institute, Cambridge, MA) and the bead-coated cells removed by magnetic separation. Typically, approximately 500 x 106 CD4[+] T cells were recovered that were >98% CD4[+] as assessed by flow cytometry and contained <0.1% monocytes as determined by staining for nonspecific esterase.

The purified CD4[+] T cells were then resuspended at 2-4 x 10$^6$ cells/ml in RPMI 1640 supplemented with 10% heat-inactivated fetal bovine serum ("medium") supplemented with 4 μg/ml PHA and 40 μCi/ml myo-2 [$^3$H]-inositol (37 MBq/ml; Amersham Corp., Arlington Heights, IL) and incubated at 37°C in 5% C0$_2$ in air. After 72 h, the cells were washed 3 times and resuspended at 5 x 10$^6$ cells/ml in "medium" supplemented with 10 mM LiCl. After a 20 min incubation, G19-4 or the G19-4/G17-2 heteroconjugate was added at t=0 to a final concentration of 1 μg/ml. Then, at various time points, samples of 5 x 10$^6$ cells were removed and sedimented for 10 sec in an Eppendorf 5414 centrifuge. After aspiration of the medium, 1 ml of ice cold 10% TCA (w/v) was added to the cellular pellet. After removal of insoluble material by 900 x g centrifugation for 5 min, the supernatant was extracted with 6 volumes of diethylether and then neutralized. The [$^3$H]-inositol phosphates were separated by anion exchange chromatography using Dowex® I-X8 in formate form (100-200 mesh) (Bio-Rad, Richmond, CA) and quantified by liquid scintillation spectroscopy in Bio-Safe 2 (Research Products, Int., Mt. Prospect, IL). This procedure for measuring tritiated inositol phosphate has been described by J. B. Imboden et al., "Transmembrane Signalling By The T Cell Antigen Receptor", supra and J.B. Imboden et al.; "Antigen Recognition By A Human T Cell Clone Leads To Increases In Inositol Trisphosphate", supra.

As Figure 4 demonstrates, at 1 $\mu$g/ml, the heteroconjugate stimulated substantial increases in the formation of inositol phosphate 1, 2 and 3 while G19-4 alone caused only a small increase in inositol phosphate 1. The tritiated inositol phosphate levels in unstimulated cells at the beginning (t=0) and end of the experiment (t=10 min) are also shown.

In additional experiments, depicted in Figure 5, we observed that free monoclonal antibody to CD4, G17-2, inhibited the inositol phosphate response caused by the heteroconjugate. G17-2 did not inhibit the inositol phosphate response to free anti-CD3 monoclonal antibody, G19-4. According to these experiments, CD4$^+$ T cells were incubated for 72 h in medium containing [$^3$H]-inositol (40 $\mu$Ci/ml) and PHA (4 $\mu$g/ml), washed extensively, and then resuspended in medium with 10 mM LiCl for a 20 min incubation at 37°C. G17-2 (biotin-conjugated as described in J.W. Goding, In Monoclonal Antibodies Principles And Practices, p. 230, Academic Press (1983)) was then added to selected samples. After 5 min, G19-4, G17-2, the G19-4/G17-2 heteroconjugate, and avidin were added as indicated, and the incubations were continued for an additional 10 min. Following lysis of the cellular samples (10$^7$ cells/sample) in 10% TCA, [$^3$H]-inositol phosphates were extracted, resolved on an ion exchange chromatography, and quantified as described above. Two independent experiments are depicted in Figure 5. The mean +/- sem (standard error) for triplicate cellular samples is shown and the final concentration in $\mu$g/ml of monoclonal antibody and avidin is given in the figure.

Thus, the experimental data demonstrates not only the enhanced effect of the heteroconjugate of the invention on inositol phosphate production but also that soluble antibody to CD4 inhibits this T cell response. The data suggests therefore the important role the CD4 antigen plays in stimulating T cell responses.

<u>The CD3/CD4 Heteroconjugate Has A Novel Activity In T Cell Activation</u>

It has been discovered that the CD3/CD4 heteroconjugate of the invention possesses a novel functional activity in T cell activation as demonstrated in proliferation assays using CD3/CD3 and CD4/CD4 homoconjugates as controls. In those assays, CD4$^+$ T cells were cultured in quadruplicate samples in flat-bottom, 96 well microtiter plates at 5 x 10$^4$ cells per well in RPMI 1640 medium containing 5% heat-inactivated fetal calf serum (Hyclone, Logan, UT). The CD4$^+$ cells were cultured for 3 days with either G19-4 (anti-CD3), G19-4/G19-4 (CD3/CD3 homoconjugate), G19-4/G17-2 (CD3/CD4 heteroconjugate) or G17-2/G17-2 (CD4/CD4 homoconjugate) with or without PMA (phorbol myristate acetate) (1 $\mu$g/ml) or monoclonal antibody 9.3, an antibody reactive with the CD28 antigen on the T cell surface [(Balb/c X C57BL/6)FI, IgG$_{2a}$] [see, e.g., J.A. Hansen et al., "Monoclonal Antibodies Identifying A Novel T Cell Antigen And Ia Antigens Of Human Lymphocytes", <u>Immunogenetics</u> 10: 247-52 (1980)]. All antibodies and conjugates were used at 1 $\mu$g/ml. Thymidine incorporation (mean cpm ± sem) was determined on day 3 in a liquid scintillation counter after pulsing the cells for the last 8 hours of the 3 day cultures with 1 $\mu$Ci/well of $^3$H-thymidine (New England Nuclear Corp., Boston, MA).

As shown in Figure 6, when the purified CD4$^+$ T cells were cultured either with the anti-CD3 reagents alone or with PMA alone, no proliferation was observed. In the presence of PMA, all antibody preparations containing monoclonal antibody to CD3 induced comparable levels of [$^3$H]-thymidine incorporation, whereas the CD4 homoconjugate was not mitogenic. When the various reagents were tested for synergy with the monoclonal antibody to CD28, 9.3, however, substantial differences were observed. Only the CD3/CD4 heteroconjugate induced responsiveness to the CD28 antibody. Previous studies have shown that CD28 stimulation is not mitogenic for purified T cells although monoclonal antibody to CD28 causes >95% of T cells to progress through the cell cycle in the presence of PMA [see, e.g., C.H. June et al., <u>Mol. Cell. Biol.</u>, <u>supra</u>]. The present finding that CD28 stimulation of purified T cells is not comitogenic with fluid phase CD3 antibodies confirms the previous studies [see, e.g., A. Weiss et al., "Synergy Between The T3/Antigen Receptor Complex And Tp44 In The Activation Of Human T Cells", <u>J. Immunol.</u>, 137 (No. 3): 819-825 (1986)].

In order to determine whether the difference between the CD3/CD4 heteroconjugate and the CD3/CD3 homoconjugate with respect to CD28 stimulation was qualitative or quantitative, CD4$^+$ T cells were cultured with G19-4, the G19-4/G17-2 heteroconjugate or the G19-4/G19-4 homoconjugate at various concentrations, with either PMA (1 $\mu$g/ml) or anti-CD28 monoclonal antibody, 9.3 (1 $\mu$g/ml). As shown in Figure 7, the G19-4/G17-2 heteroconjugate was found to be fully comitogenic with CD28 stimulation even at concentrations as low as 100 ng/ml whereas the CD3/CD3 homoconjugate was not mitogenic at 10 $\mu$g/ml. In contrast, the comitogenic response of cells to PMA and CD3 stimulation was similar for CD3 antibody conjugates at all concentrations tested. Furthermore, kinetic experiments revealed that the difference between the CD3/CD3 homoconjugate and the CD3/CD4 heteroconjugate to CD28 stimulation was not due to a shift in the time course of the proliferation (data not shown).

Thus, it appears that CD4$^+$ T cells that do not respond, by proliferation, to soluble antibody reactive with CD3, do respond to the CD3/CD4 heteroconjugate of the invention by proliferating in the presence of antibody to CD28.

To further examine the effects of the CD3/CD4 heteroconjugate on T cell proliferation, cell cycle kinetics were examined by a technique that allows discrimination of cells in each phase of three sequential cell cycles after stimulation.

CD4[+] T cells were sorted by flow cytometry on a Cytofluorograph 50 HH cell sorter (Ortho Diagnostic Systems, Inc., Westwood, MA) after staining of the cells with PE-conjugated CD8, CD16, and CD20 anti-bodies (referenced earlier). Negative cells were sorted and these were 97% CD4 positive by reanalysis of the sorted population. In addition to the immunofluorescence parameter, forward scatter and right angle scatter were measured and used to gate on the lymphocyte population and exclude monocytes, debris and dead cells. The cells were sorted with a flow rate of 2000 cells/sec at 20°C.

Sorted cells were cultured in quadruplicate in round bottom, 96 well microtiter plates (Nunc, Kampstrip, Denmark) at $5 \times 10^4$ cells/well. The G19-4/G17-2 heteroconjugate or CD3 or CD4 homoconjugates were added to the cultures at 5 μg/ml or PHA was added at 10 μg/ml. The cells were grown in RPMI 1640 supplemented with 10 FBS in the presence of 0.6 μg/ml anti-CD28 monoclonal antibody, 9.3, $1.5 \times 10^{-4}$ M BrdU, 100 U/ml penicillin and 100 μg/ml streptomycin, and $5 \times 10^{-5}$ M 2-mercaptoethanol. Three days after incubation at 37°C (5% $CO_2$), the medium was gently aspirated from the wells and replaced with 0.2 ml staining solution containing 0.1 M Tris, pH 7.4, 0.9% NaCl, 1 mM $CaCl_2$, 1.0 mM $MgCl_2$, 0.2% BSA, 0.1% NP40 and 1 μg/ml Hoechst 33258 (Sigma) and 5 μg/ml ethidium bromide, EB (Calbiochem). The cells were incubated for 30 min at 4°C, resuspended, and flow cytometry was performed on an ICP-22 (Ortho Diagnostic Systems, Inc.) interfaced to a PDP11/03 computer (Digital Equipment, Maynard, MA), as previously described by P.S. Rabinovitch, "Detection Of An Unusually Stable Fibrinolytic Inhibitor Produced By Bovine Endothelial Cells", Proc. Nat'l. Acad. Sci. USA, 80: 2956-60 (1983). Ultraviolet excitation (UGl filter) was used, with Hoechst 33258 emission detected at 425 to 500 nm and fluorescence detected above 600 nm. The fluorescence of approximately $3\text{-}4 \times 10^3$ cells was analyzed and recorded as a bivariate histogram of Hoechst versus EB fluorescence. Determination of individual cell cycle compartment sizes was by analysis and curve fitting as previously reported by P.S. Rabinovitch, id.

The percentage of cells past the first, second or third cell cycles are shown in Figure 8. As the figure demonstrates, only the CD3/CD4 heteroconjugate was able to significantly drive the cells through the cell cycle, as 45% of cells entered S phase or beyond and 39% of cells entered a second cycle of proliferation after 3 days of stimulation with the heteroconjugate. Thus, the incorporation of $^3$H-thymidine by purified CD4[+] cells in response to the CD3/CD4 heteroconjugate plus anti-CD28 stimulation (as shown in Figures 6 and 7) represents a substantial proliferative response that persisted into a second and third generation. In contrast, none of the other antibody preparations could induce significant cell cycle transition (see Figure 8). Figure 8 also indicates that, in comparison to the PHA plus anti-CD28 control, where 70% of the sorted cells had entered S phase after three days of culture, 45% of cells stimulated by the CD3/CD4 heteroconjugate had responded. This indicates that a significant number of CD4[+] cells had cell cycle progression after stimulation with the CD3/CD4 heteroconjugate.

The CD3/CD4 Heteroconjugate Comodulates The CD3 And CD4 T Cell Antigens

The modulation of CD3 and CD4 antigens on the T cell surface by the CD3/CD4 heteroconjugate, CD3 or CD4 homoconjugates or antibody to CD4 or CD3 alone was measured by determining the density of antibody or antibody conjugates on the surface of CD4[+] T cells after an 18 h incubation at 37°C in the presence of 10 μg/ml antibody or antibody conjugate, using immunofluorescence with an anti-mouse Ig second step and measured on a FACS IV flow cytometer. The results are shown in Table II below, wherein density is expressed as units of mean fluorescence intensity over background measured on a linear scale.

Table II

| Modulation By Conjugates | | | |
|---|---|---|---|
| Specificity | Surface Density Before Modulation | Surface Density After Modulation | Percent Modulation |
| CD4 | 150 | 48 | 68 |
| CD4/CD4 Homoconjugate | 226 | 75 | 67 |
| CD3 | 177 | 9.0 | 95 |
| CD3/CD3 Homoconjugate | 340 | 10 | 99 |
| CD3/CD4 Heteroconjugate | 369 | 19 | 95 |

As the table indicates, overnight incubation with antibody to CD3 or a CD3/CD3 homoconjugate resulted in an almost undetectable surface expression of CD3, whereas significant amounts of surface CD4 still remained after similar incubation with antibody to CD4 or a CD4/CD4 homoconjugate. The CD3/CD4 heteroconjugate however caused the modulation of both CD3 and CD4 and appeared to decrease the surface expression of CD4. All antibodies and conjugates used in these assays were in excess since no cell surface free CD3 or CD4 antigen (i.e., unbound by antibody) was detectable after modulation, when assayed using direct fluorescence conjugates of anti-CD3 or anti-CD4 monoclonal antibodies (data not shown). Thus, the CD3/CD4 heteroconjugate of the invention comodulates the CD3 and CD4 antigens on the surface of T cells, indicating that the heteroconjugate is not simply anchoring the T cell receptor to the cell surface as has been proposed in the case of immobilized antibody to CD3 [see, e.g., J.A. Ledbetter et al., J. Immunol., supra]. Furthermore, studies with soluble anti-CD3 have indicated that when CD3 is modulated from the cell surface, the CD3/Ti receptor is internalized [see, e.g., 0.W. Press et al., "Evaluation Of Ricin A Chain Immunotoxins Directed Against Human T Cells", Cellular Immunol., 102: 10-20 (1986) and O. W. Press et al., "Endocytosis And Degradation Of Murine Anti-Human CD3 Monoclonal Antibodies By Normal And Malignant T Lymphocytes", Cancer Research, 48:2249-2257 (1988)] leading to inhibition of T cell activation. The heteroconjugates of this invention also lead to the internalization of the receptor, yet T cell activation is enhanced.

As this example demonstrates, the CD3/CD4 heteroconjugate of this invention enhances both $Ca^{2+}$ mobilization and inositol phosphate production in the activation of $CD4^+$ T cells. In addition, the heteroconjugate possesses a novel functional activity in the proliferation of $CD4^+$ T cells in the presence of antibody to CD28, driving a significant fraction of the cells into a second cell cycle within the first three days of stimulation. Furthermore, the heteroconjugate appears to comodulate the antigens with which it is reactive, supporting the hypothesis that it acts to bring those antigens into close proximity in the activation of the T cells.

EXAMPLE 2

The following example describes the construction and characterization of other heteroconjugates of the inventtion, comprised of an antibody reactive with the CD3 antigen cross-linked to any of a number of other antibodies reactive with CD T cell antigens.

The antibody heteroconjugates, constructed as described in Example 1 above, were: a) a CD3/CD2 heteroconjugate comprising monoclonal antibodies G19-4 (anti-CD3) (referenced earlier) and 9.6 (anti-CD2), [see, e.g., P.J. Martin et al., "Identification And Functional Characterization Of Two Distinct Epitopes On The Human T Cell Surface Protein Tp50", J. Immunol., 131: 180 (1983)]; b) a CD3/CD5 heteroconjugate comprising monoclonal antibodies G19-4 and 10.2 (anti-CD5) [see, e.g., P.J. Martin et al., "A New Human T-Cell Differentiation Antigen; Unexpected Expression On Chronic Lymphocytic Leukemic Cells", Immunogenetics. 11: 429 (1980) and P.J. Martin et al., "Monoclonal Antibodies Recognizing Normal Human T Lymphocytes And Malignant B Lymphocytes A Comparative Study", J. Immunol., 12: 1920 (1981)]; c) a CD3/CD6 heteroconjugate comprising monoclonal antibodies G19-4 and G3-6 (anti-CD6) (G3-6 is an anti-CD6 monoclonal antibody of the $IgG_1$ isotype produced by immunization of Balb/c mice with the T leukemia cell line, HSB2. Hybridomas were produced by the fusion of spleen cells from the mice with the NSI myeloma line and screened by binding to normal T cells and cross-blocking with a CD6 reference antibody, 12.1 [see, e.g., P.J. Martin et al., J. Immunol., 127, supra]); d) a CD3/CD7 heteroconjugate comprising monoclonal antibodies G19-4 and G3-7 (anti-CD7) (G3-7 is an anti-CD7 monoclonal antibody of the $IgG_1$ isotype produced by I immunization of Balb/c mice with the T leukemia cell line, HSB2. Hybridomas were produced by the fusion of spleen cells from the mice with the NSI myeloma line and screened by binding to normal T cells and cross-blocking with a CD7 reference antibody, 3AI, available from the American Type Culture Collection. In addition, an anti-CD7 monoclonal antibody is commercially available (Becton Dickinson, Mountainview, CA)) [see also, e.g., E.L. Reinherz et al. (eds.), Leukocyte Typing II, Vol. 1, pp. 3-30 (1986)]; e) a CD3/CD8 heteroconjugate comprising monoclonal antibodies G19-4 and GlO-1 (anti-CD8) (referenced earlier); and f) a CD3/CD28 heteroconjugate comprising monoclonal antibodies G19-4 and 9.3 (referenced earlier).

These heteroconjugates were tested for their ability to enhance the mobilization of cytoplasmic $Ca^{2+}$ in T cells as described in Example 1. Each heteroconjugate was tested at 1 µg/ml, a concentration at which the activity of antibody to CD3 alone is suboptimal and enhanced activity of the conjugates is detected. Peak $[Ca^{2+}]i$ responses of indo-l loaded peripheral blood lymphocytes occurred within the first 5 min after stimulation of the cells with the heteroconjugate. Unstimulated cells showed a $[Ca^{2+}]i$ of 130 nM. In the experiments with the CD3/CD4 and CD3/CD8 heteroconjugates, the activity of the conjugate was measured on $CD4^+$ and $CD8^+$ T cell subsets, respectively. The activity of these heterocon-

EP 0 336 379 B1

jugates in calcium mobilization is shown in Table III below.

Table III

| Activity of Anti-CD3 Heteroconjugates in Calcium Mobilization | |
|---|---|
| Conjugate | Peak [Ca$^{2+}$] (nM) |
| CD3/CD3 (G19-4/G19-4) | 186 |
| CD3/CD2 (G19-4/9.6) | 2722 |
| CD3/CD4 (G19-4/G17-2) | 3349 |
| CD3/CD5 (G19-4/10.2) | 155 |
| CD3/CD6 (G19-4/G3-6) | 836 |
| CD3/CD7 (G19-4/G3-7) | 276 |
| CD3/CD8 (G19-4/G1O-1) | 2690 |
| CD3/CD28 (G19-4/9.3) | 204 |

As Table III indicates, the CD3/CD2, CD3/CD6, CD3/CD7 and CD3/CD8 heteroconjugates (as well as the CD3/CD4 heteroconjugate) caused a marked increase in calcium mobilization within the T cells treated with those heteroconjugates as compared to the activity of unstimulated or CD3/CD3-stimulated cells. It is believed that these heteroconjugates, like the CD3/CD4 heteroconjugate described in Example 1, act by bringing the respective antigens they react with into close proximity, resulting in enhanced transmembrane signalling and T cell activation.

EXAMPLE 3

This example describes the construction and characterization of still other heteroconjugates of the invention, comprised of an antibody reactive with the CD5 T cell surface antigen cross-linked to any of a number of other CD T cell antigens.

These antibody heteroconjugates, constructed as described above in Example 1, were the following: a) a CD5/CD2 heteroconjugate comprising monoclonal antibodies 10.2 and 9.6; b) a CD5/CD28 heteroconjugate comprising monoclonal antibodies 10.2 and 9.3; c) a CD5/CD4 heteroconjugate comprising monoclonal antibodies 10.2 and G17-2; d) a CD5/CD6 heteroconjugate comprising monoclonal antibodies 10.2 and G3-6; e) a CD5/CD7 heteroconjugate comprising monoclonal antibodies 10.2 and G3-7; and f) a CD5/CD8 heteroconjugate comprising monoclonal antibodies 10.2 and G1O-1. All the monoclonal antibodies utilized to construct the heteroconjugates of this embodiment have been referenced earlier.

These heteroconjugates were also tested for their ability to enhance the mobilization of cytoplasmic Ca$^{2+}$ in T cells as described in Example 1. The results of these experiments are shown in Table IV below. The concentration of each heteroconjugate used in these experiments is indicated in the table. Peak [Ca$^{2+}$]i responses of the indo-I loaded peripheral blood lymphocytes occurred within the first 10 min after stimulation. Unstimulated cells showed a [Ca$^{2+}$]i of 130 nM. As the table shows, a CD5/CD5 homoconjugate displayed a [Ca$^{2+}$]i of 180 nM. The CD5/CD4 and CD5/CD8 heteroconjugates were tested on CD4[+] and CD8+ T cell subsets, respectively.

Table IV

| Activity of Anti-CD5 Heteroconjugates in Calcium Mobilization | |
|---|---|
| Conjugate | Peak [$Ca^{2+}$]i (nM) |
| CD5/CD5 (10.2/10.2, 20 μg/ml) | 180 |
| CD5/CD2 (10.2/9.6, 20 μg/ml) | 204 |
| CD5/CD28 (10.2/9.3, 10 μg/ml) | 196 |
| CD5/CD4 (10.2/G17-2, 2 μg/ml) | 640 |
| CD5/CD6 (10.2/G3-6, 2 μg/ml) | 421 |
| CD5/CD7 (10.2/G3-7, 15 μg/ml) | 178 |
| CD5/CD8 (10.2/GIO-1, 2 μg/ml) | 546 |

As Table IV indicates, the CD5/CD4, CD5/CD6 and CD5/CD8 heteroconjugates show a significant increase in calcium mobilization compared with unstimulated or CD5/CD5-stimulated cells. It is believed that these heteroconjugates, like the other heteroconjugates described herein, are useful in the enhanced activation of T cells.

EXAMPLE 4

In this example a mAb-induced interaction between CD3 and CD45 antigens on the T cell surface and a heteroconjugate of antibodies reactive with CD3 and CD45 antigens were used to investigate the effect of these procedures on activation of T cells.

Cell Preparations. Peripheral blood mononuclear cells, and nylon wool nonadherent lymphocytes from peripheral blood were prepared as previously described [(Clark et al., Proc. Nat'l. Acad. Sci. USA 82:1766-1770 (1985) and Clark et al., Hum. Immunol. 16: 100-113 (1986), both incorporated by reference herein]. Briefly, nylon wool nonadherent lymphocytes were obtained by nylon wool separation as follows: a nylon column (prepared at Oncogen, Seattle, WA) was loaded with RPMI medium containing 5% fetal calf serum and was used after pre-incubation for 45 min. at 37°C. Cells were applied to the column and allowed to flow into the column and then incubated at 37°C for 45 min. Using warm media 6 mls were collected off the column, spun twice for 8 min at 1200 rpm then resuspended in proliferation buffer (15% human AB sera) (approximately 1.5 ml). The nonadherent cells were counted and the volume adjusted accordingly for use in the assays.

The following Balb/C mouse mAbs to human leukocyte markers were used: 9.4 (IgG2a), anti-CD45 [Cobold, supra]; G1-15 (IgG1) and 3AC5 (IgG2a), anti-CD45R [Ledbetter, supra and Ledbetter, et al., In Perspectives in Immunogenetics and Histocompatibility, ed. Heise, E. (ASHI, New York) pp. 325-340); 96.5 [Brown et al., J. Immunol. 127(2):639-546 (1981)]. UCHL-1 (IgG1) anti-CD45 (180 form) kindly provided by Dr. P. Beverley [Smith, supra]; G19-4 (IgG1), anti-CD3 [Ledbetter, supra]; 9.6 anti-CD2, (referenced earlier); and 9.3, anti-CD28 (referenced earlier). The rat anti-mouse kappa(κ)-specific mAb, 187.1 [Ware et al., J. Immunol. Meth. 74:93-104 (1984)] was used to cross-link mouse mAb.

Preparation of mAb Heteroconjugate. The monoclonal antibody heteroconjugates was prepared at a 1:1 molar ratio with the heterobifunctional cross-linker GMBS (Calbiochem, La Jolla, CA) and 5-iminothiolane HCl (Pierce Chemical Co., Rockford, IL) as described above in Example 1. More particularly, antibodies (1 mg/ml) were dialyzed overnight against coupling buffer (0.1 M $Na_2HPO_4$-dibasic, seven-hydrate 0.1 M NaCl, pH 7.5). The first antibody of the conjugate was thiolated with iminothiolane-HCl salt (2-IT from Pierce Chemical Co., Rockford, IL: 50 μl (0.5 mg) of 2-IT solution (10 mg/ml in coupling buffer) added while mixing. The second antibody of the conjugate was treated with GMBS: 5 μl (14 μg of GMBS solution (1 mg in 360 μl dimethylformamide (DMF)). The solutions were incubated for 1 hr at room temperature. The antibodies were run over separate PD-10 columns (Pharmacia, Uppsala, Sweden) pre-equilibrated in coupling buffer. After a void volume of 2.6 ml total, antibody was collected in double the original volume. The antibodies were mixed and incubated at room temperature for 5 hrs, and reactions were quenched by the addition of 1 μl of 25 mM β-mercaptoethanol (1 μl:560 μl coupling buffer) incubated for 15 min. at room temperature. This reaction was stopped by addition of 11 μl (11 μg) of N-ethylmaleimide (Sigma Chemical Co., St. Louis, MO) made up to 1 mg/ml in DMF.

Conjugates were dialyzed overnight against phosphate buffered saline (PBS). The resulting CD3/CD45 heteroconjugate was separated from free antibodies on Superose 6 FPLC as described in Example 1, and designated G19-4/9.4.

Conjugation of mAbs with biotin utilized biotin-succinimide (Sigma Chemical Co., St. Louis, MO) as previously described [Hardy, R. R. (1986) In Handbook of Experimental Immunology, (ed., Weir et al.), (Blackwell, Oxford) pp. 31.1-31.2 (1986)], incorporated by reference herein. Phorbol-12-myristate-13-acetate (PMA) and avidin were from Sigma Chemical Co., St. Louis, MO. Recombinant interleukin 2 (rIL2) was purchased from Genzyme (Boston, MA) and was used to promote proliferation in some assays.

Proliferation of blood T Cells was measured by incorporation of [3H]thymidine (New England Nuclear; specific activity 27 Ci/mmol; 1 ci = 37 GBq) as described above in Example 1 using $10^6$ cells per ml in 200 µl microtiter wells. More particularly, for the proliferation assays, mononuclear cells were cultured as described by Ledbetter et al., in Journal Immunol. 135:1819-1825 (1985), incorporated by reference herein. Briefly, mononuclear cells were isolated from peripheral blood by density centrifugation on Ficoll-Hypaque® followed by adherence to plastic to remove the majority of monocytes. Cells were cultured at 2.5 X $10^5$/ml in quadruplicate samples in 96-well microtiter plates (Falcon, Becton-Dickenson, Oxnard, CA) in RPMI medium containing pencillin, streptomycin, and 15% human AB serum (Pel Freez, Brown Deer, WI). Either the mitogen phytohemagglutinin (PHA; Wellcome Diagnostics, Greenville, NC) or anti-CD3 mAb (G19-4) coupled to Sepharose® was used to stimulate T cells. After 3 or 4 days in culture, wells were pulsed for 6 hr with 0.5, uCi [3H] thymidine/well (New England Nuclear, Boston, MA; 6.7 Ci/mmol specific activity). Cells were then harvested onto glass fiber filters by using a cell harvester, and radioactivity was measured in a liquid scintillation counter. In some experiments, recombinant IL2 (rIL-2; Genzyme, Boston, MA) was used at 25 U/ml. Measurements of [3H]thymidine uptake were taken during the last 6 hr. of a 3-day experiment using mAb G19-4 precoated to the wells of the microtiter plate at 10 µg/ml prior to the assay. The concentration of anti-CD45 mAb 9.4 in solution was 1 µg/µl and was immobilized on the microtiter plate at 10 mg/ml. Cells were cultured in quadruplicate and means are shown. Standard errors were <11%.

Table V summarizes the results.

Table V

| CD45 regulates T-cell proliferation After Stimulation of CD3 | | | | |
|---|---|---|---|---|
| Activation | PMA (1 ng/ml) | Proliferation ([3H]thymidine incorporation, cpm x 10-3) | | |
| | | Medium | Anti-CD45 in Solution | Anti-CD45 immobilized |
| Medium | - | 0.3 | 0.4 | 0.3 |
| | + | 0.5 | 0.5 | 0.4 |
| Anti-CD3 immobilized + rIL-2 (100 units/ml) | - | 137.9 | 143.5 | 32.3 |
| | + | 204.3 | 198.3 | 93.2 |
| | - | 196.3 | 183.9 | 67.1 |

Table V shows that anti-CD45 immobilized together with anti-CD3 inhibited T cell proliferation by more than 75%, whereas anti-CD45 in solution did not. The inhibitory effect of immobilized anti-CD45 was still apparent even in the presence of a suboptimal concentration of PMA or exogenous rIL2.

Proliferation of T cells after stimulation with anti-CD3 mAb (G19.4) was also measured with immobilized anti-CD3 mAb and CD45R mAbs (G1-15 and 3AC5) immobilized or in solution. Proliferation was measured as described above for anti-CD45. Anti-CD45R mAbs G1-15 and 3AC5 were used at 1 µg/ml in solution and immobilized at 10 µg/ml. A control IgG2a mAb 96.5 against melanoma antigen p97 was used as a nonreacting control at 1 µg/ml in solution and

10 μg/ml immobilized. Table VI shows the results of this experiment.

Table VI

| CD45 regulates T-cell proliferation After stimulation of CD3 | | | |
|---|---|---|---|
| Activation | Proliferation ([$^3$H]thymidine incorporation, cpm x 10-3) | | |
| | Medium | PMA (1 ng/ml) | rIL-2 100 units/ml |
| Medium | 0.1 | 0.5 | 0.4 |
| Anti-CD3 immobilized | | | |
| + medium | 143.6 | 281.5 | 291.8 |
| +0.-15 anti-CD45R (soln) | 137.5 | 261.5 | 309.4 |
| +3AC5 anti-CD45R (soln) | 177.8 | 266.2 | 294.6 |
| +96.5 (soln) | 147.9 | 262.1 | 312.4 |
| Anti-CD3 immobilized | | | |
| +G1-15 anti-CD45R (immobilized) | 0.1 | 0.7 | 0.6 |
| +3ACS anti-CD45R (immobilized) | 10.1 | 68.2 | 95.5 |
| +86.5 (immobilized) | 154.4 | 247.1 | 280.4 |

Inhibition was evident when either CD45R (3AC5) or CD45R (G1-15) mAbs were immobilized together with anti-CD3, but not when the CD45R mAbs were in solution. Again, neither PMA nor rIL2 could overcome this inhibition. The control mAb, 96.5, against the melanoma antigen p97, did not inhibit proliferation in response to immobilized anti-CD3 when either present in solution or immobilized together with anti-CD3.

To ensure that the inhibition seen with immobilized CD45 mAb (9.4) was not due simply to a displacement of the CD3 mAb (G19-4) from the plastic surface, microtiter wells were coated with anti-CD3 alone, anti-CD3 plus anti-CD45, or anti-CD3 plus the control mAb 96.5. Peripheral blood mononuclear cells were stimulated with anti-CD3 mAb G19-4 immobilized to the walls of a microtiter plate by incubation at room temperature in phosphate buffered saline for 2 hr. at concentrations of 4, 8, 16 and 32 μg/ml, either alone or together with anti-CD45 mAb 9.4 or with a control mAb 96.5 at the same concentration as the anti-CD3 mAb. Bovine serum albumin was added to permit further protein attachment. Proliferation with CD45 mAb 9.4 in solution was used for comparison. Proliferation was measured in the absence or presence of PMA at 1 ng/ml. Cells were cultured in quadruplicate for 3 days and pulsed with [$^3$H]thymidine during the last 6 hr. Figure 9 presents the results of these experiments (means are shown and standard errors were less than 15% of the mean at each point).

Figure 9 shows that (a) as more anti-CD3 was used for coating the wells, there was more proliferation, consistent with previous reports (Geppert et al., J. Immunol. 138: 1660-1666 (1987)); (b) the CD45 mAb 9.4 caused substantial inhibition at all concentrations, but only when immobilized; and (c) there was essentially no inhibition caused by the simultaneous coating with the isotype control 96.5 mAb, indicating that the inhibition by anti-CD45 was not the result of a displacement of anti-CD3. Addition of 1 ng/ml PMA to the cultures reversed the inhibition by immobilized anti-CD45 only when the mAb was coated at 4 μg/ml or less, but not a higher concentrations (Fig. 9B). This suggests that a critical concentration of anti-CD45 may need to be in close proximity to anti-CD3 on the plastic surface to maintain inhibition in the presence of PMA.

T cell activation resulting in proliferation can proceed by stimulation of CD28 and CD2 using mAbs 9.3 and 9.6 in solution, respectively, with subsequent cross-linking in a second step using anti-κ mAb 187.1 [Van Lier et al., Eur. J. Immunol. 18:167-175 (1988) and Ledbetter et al., Proc. Nat'l Acad. Sci. USA 84:1384-1388 (1987)]. To investigate the effects of CD45 ligation in this system, anti-CD45 was added to anti-CD28 or to anti-CD2 in solution with or without the cross-linking mAb 187.1. [Ware et al., supra]. Proliferation was measured by [$^3$H]thymidine uptake as described above: Anti-CD28 mAb 9.3 and anti-CD2 mAb 9.6 were used-for activation at 1 μg/ml each. Anti-CD45 mAb 9.4 was used at 1 μg/ml and anti-κ mAb 187.1 was used to cross-link the antibodies at a 4:1 final ratio of mAb 187.1 to mouse mAb. Proliferation was measured in the presence (100 Units/ml) and absence of rIL-2. Table VII shows the results (standard errors were <12% of the mean at each point).

Table VII

| CD45 regulates T-cell proliferation After CD2 and CD28 Simulation | | | | | |
|---|---|---|---|---|---|
| Stimulus | (100 units/ml) | Proliferation ([³H]thymidine incorporation, cpm x 10-3) | | | |
| | | Medium | mAb 187.1 | Anti-CD45 | Anti-CD45 + mAb 187.1 |
| Medium | - | 0.2 | 0.3 | 1.6 | 0.4 |
| | + | 6.8 | 4.2 | 37.6 | 2.5 |
| Anti-CD28 | - | 0.2 | 4.3 | 47.4 | 0.4 |
| | + | 12.6 | 36.1 | 72.5 | 9.6 |
| Anti-CD2 | - | 0.5 | 0.2 | 12.7 | 0.2 |
| | + | 4.4 | 7.0 | 37.3 | 4.0 |

It is apparent that addition of anti-CD45 alone could promote cell proliferation or increase that induced by either CD2 or CD28 mAbs without requiring exogenous rIL2 (Table VII). In all instances, addition of rIL2 further augmented cell proliferation even in the absence of other stimulants. These phenomena may be due to an effect of CD45 ligation on expression of high affinity receptors for IL2 (Ledbetter, supra).

In contrast, cross-linking CD45 to CD2 or CD28 by addition of the 187.1 anti-κ mAb reversed the stimulatory effect of CD45 alone and reduced the stimulation by CD2 or CD28 (Table VII) in either the presence or absence of rIL2. This was not due to a nonspecific inhibitory activity of 187.1 since CD28 or CD2 stimulation were both enhanced by 187.1 when the CD45 mAb 9.4 was not included.

CD3, CD2, and CD28 are coupled to signal transduction pathways that result in an increase in $[Ca^{2+}]i$ when these receptors are ligated on the cell surface. [Ledbetter et al., Proc. Nat'l. Acad. Sci. USA 84:1384-1388 (1987)]. $[Ca^{2+}]i$ was measured after cross-linking the CD3, CD2 or CD28 receptors in the presence or absence of CD45 mAb 9.4 (Fig. 10). In this experiment, biotin-conjugated mAbs were bound to peripheral blood T cells and then crosslinked on the cell surface by the addition of avidin. Increases in $[Ca^{2+}]i$ in peripheral blood mononuclear T cells loaded with indo-I (Molecular Probes, Junction City, OR) were measured after cross-linking receptors on T cells either along or together with anti-CD45 mAb 9.4 or anti-CD45R mAb 3AC5 as follows (conditions are set forth with reference to Figure 10):

1. the CD3 receptor was cross-linked with biotin-conjugated anti-CD3 mAb G19.4 (2 µg/ml) at time = 1.5 min., followed by avidin (8 µg/ml) added at time = 5.5 min. (—). This was compared with the same cross-linking of the CD3 receptor in the presence of anti-CD45 mAb 9.4 (10 µg/ml) (----) or to biotin-conjugated anti-CD45 mAb 9.4 (10 µg/ml) followed by avidin (48 µg) (·····). An additional control of biotin-conjugated anti-CD45 mAb 9.4 (10 µg/ml) followed by avidin (40 µg/ml) was also used (----) (Fig. 10A);

2. the effect of 25 µg/ml of anti-CD3 mAb G19.4 (—) was compared to 50 µg/ml of a CD3/CD45 heteroconjugate of anti-CD3 mAb G19-4 and anti-CD45 mAb 9.4 (·····) (Fig. 10B);

3. the CD2 receptor was cross-linked with 10 µg/ml of biotin-conjugated anti-CD2 mAb 9.6 added at time = -3 min., followed by addition of 40 µg/ml of avidin at time = 1.5 min. (—) (Fig. 10C);

4. the CD28 receptor was cross-linked with 10 µg/ml of biotin-conjugated anti-CD28 mAb 9.3 added at time = -3 min., followed by addition of 40 µg/ml of avidin at time = -3 min. (—) compared with simultaneous cross-linking of CD28 and CD45 receptors using 10 µg/ml of anti-CD28 mAb 9.3 and 10 µg/ml anti-CD45 mAb 9.4 added at time = -3 min. followed by addition of 80 µg/ml of avidin at time = 1.5 min. (····) (Fig. 10D); and

5. the CD4 receptor was cross-linked by addition of 10 µg/ml of biotin-conjugated anti-CD4 mAb G17-2 at time = -3 min., followed by 40 µg/ml of avidin added at time = 1.5 min. (—). This was compared with simultaneous cross-linking of the CD4 and CD45 receptors by addition of 10 µg/ml of biotin-conjugated anti-CD4 mAb G17-2 and 10 µg/ml of biotin-conjugated anti-CD45 mAb 9.4 at time = -3 min., followed by addition of 80 µg/ml of avidin at time = 1.5 min. (····). The comparison of biotin-conjugated anti-CD4 mAb G17-2 (10 µg/ml) plus anti-CD45 mAb 9.4 (10 µg/ml) (not conjugated) followed by 40 µg/ml of avidin at time = 1.5 min. is also shown (—·—) (Fig. 10E).

Figure 10A-E presents the results of these experiments.

It was apparent that the increase in $(Ca^{2+})i$ seen after CD3, CD2 and CD28 crosslinking was inhibited by the presence of biotin conjugated 9.4. When 9.4 was not biotinylated, and thus not crosslinked by avidin, a small decrease in the extent of anti-CD3 induced calcium mobilization was still observed (Fig. 10A). Complete inhibition, however, appeared to require that CD45 and CD3 be brought into close proximity. Furthermore, a heteroconjugate of anti-CD45 and anti-CD3 was unable to directly increase $[Ca^{2+}]i$ (Fig. 10B) even though immunofluoresence assays with an anti-mouse immunoglobulin demonstrated that the heteroconjugate could bind to both receptors on the cell surface (data not shown) and 25 µg of anti-CD3 could elicit a strong calcium response. CD45R mAbs such as 3AC5 were able to partially inhibit $[Ca^{2+})i$ responses when ligated to mAbs reactive with the cell receptors, such as illustrated for CD2 (Fig. 10C). The partial inhibition afforded by CD45R mAb may reflect the expression of CD45R isoform on some but not all T cells (Ledbetter, supra). In contrast to the inhibitory effects on CD3, CD2 and CD28, ligation of CD45 to CD4 gave a strong and reproducible augmentation of signalling as compared to when CD4 is crosslinked to itself (Fig. 10E). This occurred without an increase in the number of responding cells, showing that the same CD4 positive cells were more responsive following ligation of CD45 and CD4. Thus, CD45 appears to either up or down-regulate transmembrane signalling depending on the receptor with which it is interacting.

Previous studies of CD45 suggested that it may function to either up- or down-regulate lymphocyte activity (Ledbetter, supra; Harp, supra and Martorell, supra). Soluble mAbs to CD45 or CD45R are co-stimulatory with PHA or anti-CD3 attached to beads in increasing IL-2 production, IL-2 receptor expression, and T-cell proliferation. Soluble anti-CD45 can act cooperatively with CD2 or CD28 mAb (Table VI). These co-stimulatory effects are restricted to CD4+ cells and not seen with CD8+ cells which do not express CD4. This may be due in part to the fact that CD45 has a characteristic effect on CD4 distinct from its action on other cell surface, antigens such as CD3 or CD2; when CD45 is brought into close association with CD4, it acts to accelerate and increase the calcium signal transmitted via CD4 while inhibiting signal transduction when coupled to CD3 and CD2 (Fig. 10).

The above example (4) demonstrates that the inhibitory effects of anti-CD45 are most clearly demonstrated under conditions that place CD45 into close contact with signal transducing elements such as the T cell receptors CD2, CD3, CD5 or CD28 as demonstrated by use of the CD4/CD45 heteroconjugate of the invention. The anti-CD45 antigen can act very early in lymphocyte activation, inhibiting the mobilization of intracellular free calcium normally detectable within 30 to 60 sec. The enhancing effects of anti-CD45 are also demonstrated by placing the CD45 receptor into proximity with the CD4 receptor. These results suggest that heteroconjugates containing molecules reactive with receptors on lymphocytes can provide an alternative method for inducing interaction between the receptors to enhance or inhibit activation and function of the lymphocytes, including T cells and B cells.

While we have hereinbefore presented a number of embodiments of the invention, it is apparent that the basic construction of this invention can be altered to provide still other embodiments which lead to the enhanced activation of lymphocytes, e.g., other lymphocyte antigen heteroconjugate combinations or to the inhibition of the function of lymphocytes. Therefore, it will be appreciated that the scope of this invention is to be defined by the claims appended hereto rather than by the specific embodiments which have been presented hereinbefore by way of example.

## Claims

**Claims for the following Contracting States : AT, BE, CH, DE, FR, GB, IT, LI, LU, NL, SE**

1. A heteroconjugate useful in the regulation of lymphocytes comprising at least two molecules selected from antibodies and ligand peptides capable of binding to antigens on lymphocytes, said molecules being cross-linked to each other, each molecule being reactive with a different antigen located on the surface of individual lymphocytes.

2. The heteroconjugate of claim 1, wherein the heteroconjugate is an antibody heteroconjugate useful in the activation of T-lymphocytes comprising at least two antibody molecules cross-linked to each other, each antibody being reactive with a different antigen located on the surface of T lymphocytes.

3. The heteroconjugate of claim 1 or 2, wherein the antigen is a CD antigen on the surface of a T lymphocyte.

4. The heteroconjugate of claim 1, wherein the antigen is a CD antigen on the surface of a B lymphocyte.

5. The heteroconjugate of claim 1 or 2, wherein one of the molecules of the heteroconjugate is reactive with the T cell receptor or its associated CD3 antigen.

6. The heteroconjugate of claim 1 or 2, wherein one of the molecules of the heteroconjugate is reactive with the CD5 T-cell surface antigen.

7. The heteroconjugate of claim 5, being a CD3/CD4 antibody heteroconjugate.

8. The heteroconjugate of claim 7, being antibody heteroconjugate G19-4/G17-2.

9. The heteroconjugate of claim 5, being an antibody heteroconjugate selected from the group consisting of CD3/CD2, CD3/CD6, CD3/CD7 and CD3/CD8.

10. The heteroconjugate of claim 6, being an antibody heteroconjugate selected from the group consisting of CD5/CD4, CD5/CD6 and CD5/CD8.

11. The heteroconjugate of claim 1 or 2, wherein one of the molecules of the heteroconjugate is reactive with CD45 antigen or isoforms of the CD45 antigen.

12. The heteroconjugate of claim 11, being antibody heteroconjugate CD3/CD45.

13. The heteroconjugate of claim 12, being antibody heteroconjugate G19-4/9.4.

14. The heteroconjugate of claim 11, being an antibody heteroconjugate selected from the group consisting of CD2/CD45, CD4/CD45 and CD28/CD45.

15. The heteroconjugate of claim 11, being an antibody heteroconjugate selected from the group consisting of CD2/CD45R, CD3/CD45R, CD4/CD45R and CD28/CD45R.

16. The heteroconjugate of claim 2, wherein the antibodies are comprised of antibody fragments selected from the group consisting of Fab and F(ab')$_2$ fragments.

17. The antibody heteroconjugate of claim 2, wherein the antibodies are monoclonal.

18. The heteroconjugate of claim 2, wherein the antibodies are chimeric.

19. The use of at least one heteroconjugate according to claim 2 for preparing a pharmaceutical composition for activating T lymphocytes.

20. The use of the heteroconjugate of claim 1 or 2 for preparing a pharmaceutical composition for regulating the function of T lymphocytes and/or B lymphocytes.

21. The use according to claim 20, wherein at least one of the antibodies of the heteroconjugate is reactive with the CD45 cell surface antigen or an isoform of the CD45 antigen.

22. An in vitro method for treating disease comprising the step of activating T lymphocytes in vitro by contacting the lymphocytes with at least one heteroconjugate according to claim 1.

23. The method of claim 22, wherein the disease is selected from the group consisting of infectious disease, cancer, AIDS and autoimmune disease.

24. A pharmaceutically acceptable composition useful in the treatment of disease which comprises a pharmaceutically effective amount of at least one heteroconjugate according to anyone of claims 1 to 18.

25. The use of at least one heteroconjugate according to anyone of claims 1 to 18 for preparing a pharmaceutical composition for treating disease, in particular infectious disease, cancer, AIDS or autoimmune disease.

26. The in vitro use of at least one heteroconjugate according to anyone of claims 1 to 18 for diagnosing a defect in CD receptor function in patients suffering from a disease involving T cell disregulation or dysfunction comprising the step of measuring calcium mobilization activity of the heteroconjugate on the T lymphocytes of that patient compared to the activity of the unconjugated antibodies that make up the components of the heteroconjugate.

27. The use of a heteroconjugate of claim 7 or 11 for preparing a pharmaceutical composition to selectively regulate subpopulations of T and B lymphocytes in the treatment of autoimmune disease.

**28.** A method of preparing heteroconjugates of claim 1 useful in the regulation of lymphocytes comprising cross-linking at least two molecules capable of binding to antigens on lymphocytes, each molecule reactive with a different antigen on the surface of individual lymphocytes.

**29.** The method of claim 28, wherein said lymphocytes with which said molecules are reactive are T lymphocytes.

**30.** The method of claim 28, wherein the antigen is a CD antigen on the surface of a T lymphocyte.

**31.** The method of claim 28, wherein the antigen is a CD antigen on the surface of a B lymphocyte.

**32.** The method of claim 28, wherein one of the molecules of the heteroconjugate is reactive with the T cell receptor or its associated CD3 antigen.

**33.** The method of claim 28, wherein one of the molecules of the heteroconjugate is reactive with the CD5 T cell surface antigen.

**34.** The method of claim 28 or 29, wherein said molecules are the antibody reactive with CD3 antigen and the antibody reactive with CD4 antigen.

**35.** The method of claim 34, wherein said antibodies are the antibody G19-4 crosslinked to the antibody G17-2.

**36.** The method of claim 28, wherein said molecules are an antibody reactive with the CD3 antigen and an antibody selected from the group consisting of antibodies reactive with CD6, CD7 and CD8 antigen.

**37.** The method of claim 28, wherein said molecules are an antibody reactive with the CD5 antigen and an antibody selected from the group consisting of antibodies reactive with CD4, CD6 and CD8 antigen.

**38.** The method of claim 28, wherein one of the molecules is reactive with CD45 antigen or isoforms of the CD45 antigen.

**39.** The method of claim 28, wherein one of the molecules is an antibody selected from the group consisting of antibodies reactive with CD2, CD3, CD4 and CD28 antigen, and the other molecule is an antibody reactive with the CD45 antigen.

**40.** The method of claim 28, wherein one of the molecules is an antibody selected from the group consisting of antibodies reactive with CD2, CD3, CD4 and CD 28 antigen, and the other molecule is an antibody reactive with the CD45R antigen.

**41.** The method of any one of claims 28-40, wherein said molecules are monoclonal antibodies.

**42.** The method of claim 28, wherein the molecules are comprised of antibody fragments selected from the group consisting of Fab and $F(ab')_2$ fragments.

**43.** The method of claim 28, wherein the molecules are chimeric antibodies.

**44.** The method of claim 28, wherein said cross-linking is chemical cross-linking.

**45.** The method of claim 44, wherein said chemical cross-linking is performed using a reagent selected from the group consisting of maleimidobutryloxy succinimide and N-succinimidyl 3-(2-pyridyldithio)propionate.

**46.** An in vitro method for diagnosing a defect in CD receptor function in patients suffering from a disease involving T cell disregulation or dysfunction comprising the step of measuring calcium mobilization activity of a heteroconjugate according to anyone of claims 1 or 2 on the T lymphocytes of that patient compared to the activity of the unconjugated antibodies that make up the components of the heteroconjugate.

**Claims for the following Contracting State : ES**

**1.** A method of preparing a heteroconjugate useful in the regulation of lymphocytes comprising at least two molecules selected from antibodies and ligand peptides capable of binding to antigens on lymphocytes, each molecule being reactive with a different antigen located on the surface of individual lymphocytes, which method comprises cross-linking said molecules to each other.

2. The method of claim 1, wherein said lymphocytes with which said molecules are reactive are T lymphocytes.

3. The method of claim 1 or 2, wherein the heteroconjugate is an antibody heteroconjugate useful in the activation of T lymphocytes comprising at least two antibody molecules cross-linked to each other, each antibody being reactive with a different antigen located on the surface of T lymphocytes.

4. The method of claim 1, 2 or 3, wherein the antigen is a CD antigen on the surface of a T lymphocyte.

5. The method of claim 1, wherein the antigen is a CD antigen on the surface of a B lymphocyte.

6. The method of claim 1, 2 or 3, wherein one of the molecules of the heteroconjugate is reactive with the T cell receptor or its associated CD3 antigen.

7. The method of claim 1, 2 or 3, wherein one of the molecules of the heteroconjugate is reactive with the CD5 T-cell surface antigen.

8. The method of claim 6, wherein a CD3/CD4 antibody heteroconjugate is prepared.

9. The method of claim 8, wherein antibody heteroconjugate G19-4/G17-2 is prepared.

10. The method of claim 6, wherein an antibody heteroconjugate selected from the group consisting of CD3/CD2, CD3/CD6, CD3/CD7 and CD3/CD8 is prepared.

11. The method of claim 7, wherein an antibody heteroconjugate selected from the group consisting of CD5/CD4, CD5/CD6 and CD5/CD8 is prepared.

12. The method of claim 1, 2 or 3, wherein one of the molecules of the heteroconjugate is reactive with CD45 antigen or isoforms of the CD45 antigen.

13. The method of claim 12, wherein antibody heteroconjugate CD3/CD45 is prepared.

14. The method of claim 13, wherein antibody heteroconjugate G19-4/9.4 is prepared.

15. The method of claim 12, wherein an antibody heteroconjugate selected from the group consisting of CD2/CD45, CD4/CD45 and CD28/CD45 is prepared.

16. The method of claim 12, wherein an antibody heteroconjugate selected from the group consisting of CD2/CD45R, CD3/CD45R, CD4/CD45R and CD28/CD45R is prepared.

17. The method of one of the preceding claims, wherein the antibodies are comprised of antibody fragments selected from the group consisting of Fab and F(ab')$_2$ fragments.

18. The method of one of the preceding claims, wherein the antibodies are monoclonal.

19. The method of one of the preceding claims, wherein the antibodies are chimeric.

20. The method of one of the preceding claims, wherein said chemical cross-linking is performed using a reagent selected from the group consisting of maleimidobutryloxy succinimide and N-succinimidyl 3-(2-pyridyldithio)propionate.

21. The use of at least one heteroconjugate according to claim 3 for preparing a pharmaceutical composition for activating T lymphocytes.

22. The use of the heteroconjugate of claim 1, 2 or 3 for preparing a pharmaceutical composition for regulating the function of T lymphocytes and/or B lymphocytes.

EP 0 336 379 B1

23. The use according to claim 22, wherein at least one of the antibodies of the heteroconjugate is reactive with the CD45 cell surface antigen or an isoform of the CD45 antigen.

24. An in vitro method for treating disease comprising the step of activating T lymphocytes in vitro by contacting the lymphocytes with at least one heteroconjugate according to claim 1.

25. The method of claim 24, wherein the disease is selected from the group consisting of infectious disease, cancer, AIDS and autoimmune disease.

26. A method of preparing a pharmaceutically acceptable composition useful in the treatment of disease which method comprises providing a pharmaceutically effective amount of at least one heteroconjugate prepared according to anyone of claims 1 to 20.

27. The use of at least one heteroconjugate prepared according to anyone of claims 1 to 20 for preparing a pharmaceutical composition for treating disease, in particular infectious disease, cancer, AIDS or autoimmune disease.

28. The in vitro use of at least one heteroconjugate prepared according to anyone of claims 1 to 20 for diagnosing a defect in CD receptor function in patients suffering from a disease involving T cell disregulation or dysfunction comprising the step of measuring calcium mobilization activity of the heteroconjugate on the T lymphocytes of that patient compared to the activity of the unconjugated antibodies that make up the components of the heteroconjugate.

29. The use of a heteroconjugate of claim 8 or 12 for preparing a pharmaceutical composition to selectively regulate subpopulations of T and B lymphocytes in the treatment of autoimmune disease.

30. The in vitro method for diagnosing a defect in CD receptor function in patients suffering from a disease involving T cell disregulation or dysfunction comprising the step of measuring calcium mobilization activity of a heteroconjugate according to anyone of claims 1, 2 or 3 on the T lymphocytes of that patient compared to the activity of the unconjugated antibodies that make up the components of the heteroconjugate.

**Claims for the following Contracting State : GR**

1. A heteroconjugate useful in the regulation of lymphocytes comprising at least two molecules selected from antibodies and ligand peptides capable of binding to antigens on lymphocytes, said molecules being cross-linked to each other, each molecule being reactive with a different antigen located on the surface of individual lymphocytes.

2. The heteroconjugate of claim 1, wherein the heteroconjugate is an antibody heteroconjugate useful in the activation of T-lymphocytes comprising at least two antibody molecules cross-linked to each other, each antibody being reactive with a different antigen located on the surface of T lymphocytes.

3. The heteroconjugate of claim 1 or 2, wherein the antigen is a CD antigen on the surface of a T lymphocyte.

4. The heteroconjugate of claim 1, wherein the antigen is a CD antigen on the surface of a B lymphocyte.

5. The heteroconjugate of claim 1 or 2, wherein one of the molecules of the heteroconjugate is reactive with the T cell receptor or its associated CD3 antigen.

6. The heteroconjugate of claim 1 or 2, wherein one of the molecules of the heteroconjugate is reactive with the CD5 T-cell surface antigen.

7. The heteroconjugate of claim 5, being a CD3/CD4 antibody heteroconjugate.

8. The heteroconjugate of claim 7, being antibody heteroconjugate G19-4/G17-2.

9. The heteroconjugate of claim 5, being an antibody heteroconjugate selected from the group consisting of CD3/CD2, CD3/CD6, CD3/CD7 and CD3/CD8.

10. The heteroconjugate of claim 6, being an antibody heteroconjugate selected from the group consisting of CD5/CD4, CD5/CD6 and CD5/CD8.

11. The heteroconjugate of claim 1 or 2, wherein one of the molecules of the heteroconjugate is reactive with CD45 antigen or isoforms of the CD45 antigen.

12. The heteroconjugate of claim 11, being antibody heteroconjugate CD3/CD45.

13. The heteroconjugate of claim 12, being antibody heteroconjugate G19-4/9.4.

14. The heteroconjugate of claim 11, being an antibody heteroconjugate selected from the group consisting of CD2/CD45, CD4/CD45 and CD28/CD45.

15. The heteroconjugate of claim 11, being an antibody heteroconjugate selected from the group consisting of CD2/CD45R, CD3/CD45R, CD4/CD45R and CD28/CD45R.

16. The heteroconjugate of claim 2, wherein the antibodies are comprised of antibody fragments selected from the group consisting of Fab and F(ab')$_2$ fragments.

17. The antibody heteroconjugate of claim 2, wherein the antibodies are monoclonal.

18. The heteroconjugate of claim 2, wherein the antibodies are chimeric.

19. The use of at least one heteroconjugate according to claim 2 for preparing a pharmaceutical composition for activating T lymphocytes.

20. The use of the heteroconjugate of claim 1 or 2 for preparing a pharmaceutical composition for regulating the function of T lymphocytes and/or B lymphocytes.

21. The use according to claim 20, wherein at least one of the antibodies of the heteroconjugate is reactive with the CD45 cell surface antigen or an isoform of the CD45 antigen.

22. An in vitro method for treating disease comprising the step of activating T lymphocytes in vitro by contacting the lymphocytes with at least one heteroconjugate according to claim 1.

23. The method of claim 22, wherein the disease is selected from the group consisting of infectious disease, cancer, AIDS and autoimmune disease.

24. A method of preparing a pharmaceutically acceptable composition useful in the treatment of disease which method comprises providing a pharmaceutically effective amount of at least one heteroconjugate according to anyone of claims 1 to 18.

25. The use of at least one heteroconjugate according to anyone of claims 1 to 18 for preparing a pharmaceutical composition for treating disease, in particular infectious disease, cancer, AIDS or autoimmune disease.

26. The in vitro use of at least one heteroconjugate according to anyone of claims 1 to 18 for diagnosing a defect in CD receptor function in patients suffering from a disease involving T cell disregulation or dysfunction comprising the step of measuring calcium mobilization activity of the heteroconjugate on the T lymphocytes of that patient compared to the activity of the unconjugated antibodies that make up the components of the heteroconjugate.

27. The use of a heteroconjugate of claim 7 or 11 for preparing a pharmaceutical composition to selectively regulate subpopulations of T and B lymphocytes in the treatment of autoimmune disease.

28. A method of preparing heteroconjugates of claim 1 useful in the regulation of lymphocytes comprising cross-linking at least two molecules capable of binding to antigens on lymphocytes, each molecule reactive with a different antigen on the surface of individual lymphocytes.

29. The method of claim 28, wherein said lymphocytes with which said molecules are reactive are T lymphocytes.

30. The method of claim 28, wherein the antigen is a CD antigen on the surface of a T lymphocyte.

31. The method of claim 28, wherein the antigen is a CD antigen on the surface of a B lymphocyte.

32. The method of claim 28, wherein one of the molecules of the heteroconjugate is reactive with the T cell receptor or its associated CD3 antigen.

33. The method of claim 28, wherein one of the molecules of the heteroconjugate is reactive with the CD5 T cell surface antigen.

34. The method of claim 28 or 29, wherein said molecules are the antibody reactive with CD3 antigen and the antibody reactive with CD4 antigen.

35. The method of claim 34, wherein said antibodies are the antibody G19-4 crosslinked to the antibody G17-2.

36. The method of claim 28, wherein said molecules are an antibody reactive with the CD3 antigen and an antibody selected from the group consisting of antibodies reactive with CD6, CD7 and CD8 antigen.

37. The method of claim 28, wherein said molecules are an antibody reactive with the CD5 antigen and an antibody selected from the group consisting of antibodies reactive with CD4, CD6 and CD8 antigen.

38. The method of claim 28, wherein one of the molecules is reactive with CD45 antigen or isoforms of the CD45 antigen.

39. The method of claim 28, wherein one of the molecules is an antibody selected from the group consisting of antibodies reactive with CD2, CD3, CD4 and CD28 antigen, and the other molecule is an antibody reactive with the CD45 antigen.

40. The method of claim 28, wherein one of the molecules is an antibody selected from the group consisting of antibodies reactive with CD2, CD3, CD4 and CD 28 antigen, and the other molecule is an antibody reactive with the CD45R antigen.

41. The method of any one of claims 28-40, wherein said molecules are monoclonal antibodies.

42. The method of claim 28, wherein the molecules are comprised of antibody fragments selected from the group consisting of Fab and $F(ab')_2$ fragments.

43. The method of claim 28, wherein the molecules are chimeric antibodies.

44. The method of claim 28, wherein said cross-linking is chemical cross-linking.

45. The method of claim 44, wherein said chemical cross-linking is performed using a reagent selected from the group consisting of maleimidobutryloxy succinimide and N-succinimidyl 3-(2-pyridyldithio)propionate.

46. An in vitro method for diagnosing a defect in CD receptor function in patients suffering from a disease involving T cell disregulation or dysfunction comprising the step of measuring calcium mobilization activity of a heteroconjugate according to anyone of claims 1 or 2 on the T lymphocytes of that patient compared to the activity of the unconjugated antibodies that make up the components of the heteroconjugate.

**Patentansprüche**

**Patentansprüche für folgende Vertragsstaaten : AT, BE, CH, DE, FR, GB, IT, LI, LU, NL, SE**

1. Heterokonjugat zur Regulation von Lymphozyten, umfassend wenigstens zwei Moleküle, ausgewählt unter Antikörpern und Peptidliganden, welche zur Bindung an Antigenen auf Lymphozyten befähigt sind, wobei die Moleküle miteinander quervernetzt sind, wobei jedes Molekül mit einem anderen Antigen auf der Oberfläche einzelner Lymphozyten reagiert.

2. Heterokonjugat nach Anspruch 1, worin das Heterokonjugat ein Antikörper-Heterokonjugat zur Aktivierung von T-Lymphozyten ist, das wenigstgens zwei Antikörpermoleküle umfaßt, die miteinander quervernetzt sind, wobei jeder Antikörper mit einem anderen Antigen auf der Oberfläche von T-Lymphozyten reagiert.

3. Heterokonjugat nach Anspruch 1 oder 2, worin das Antigen ein CD-Antigen auf der Oberfläche eines T-Lymphozyten ist.

4. Heterokonjugat nach Anspruch 1, worin das Antigen ein CD-Antigen auf der Oberfläche eines B-Lymphozyten ist.

5. Heterokonjugat nach Anspruch 1 oder 2, worin eines der Moleküle des Heterokonjugates mit dem T-Zellrezeptor oder dessen assoziierten CD3-Antigen reagiert.

6. Heterokonjugat nach Anspruch 1 oder 2, worin eines der Moleküle des Heterokonjugates mit dem T-Zelloberflächenantigen CD5 reagiert.

7. Heterokonjugat nach Anspruch 5, nämlich das Antikörperheterokonjugat CD3/CD4.

8. Heterokonjugat nach Anspruch 7, nämlich das Antikörperheterokonjugat G19-4/G17-2.

9. Heterokonjugat nach Anspruch 5, nämlich ein Heterokonjugat, ausgewählt unter CD3/CD2, CD3/CD6, CD3/CD7 und CD3/CD8.

10. Heterokonjugat nach Anspruch 6, nämlich ein Heterokonjugat, ausgewählt unter CD5/CD4, CD5/CD6 und CD5/CD8.

11. Heterokonjugat nach Anspruch 1 oder 2, worin eines der Moleküle des Heterokonjugates mit dem CD45 Antigen oder Isoformen des CD45 Antigens reagiert.

12. Heterokonjugat nach Anspruch 11, nämlich das Antikörperheterokonjugat CD3/CD45.

13. Heterokonjugat nach Anspruch 12, nämlich das Antikörperheterokonjugat G19-4/9.4.

14. Heterokonjugat nach Anspruch 11, nämlich ein Antikörperheterokonjugat, ausgewählt unter CD2/CD45, CD4/ CD45 und CD28/CD45.

15. Heterokonjugat nach Anspruch 11, nämlich ein Heterokonjugat, ausgewählt unter CD2/CD45R, CD3/CD45R, CD4/CD45R und CD28/ CD45R.

16. Heterokonjugat nach Anspruch 2, worin die Antikörper Antikörperfragmente umfassen, die ausgewählt sind unter Fab- und F(ab')$_2$-Fragmenten.

17. Antikörperheterokonjugat nach Anspruch 2, worin die Antikörper monoklonal sind.

18. Heterokonjugat nach Anspruch 2, worin die Antikörper chimär sind.

19. Verwendung wenigstens eines Heterokonjugates nach Anspruch 2 zur Herstellung eines pharmazeutischen Mittels zur Aktivierung von T-Lymphozyten.

20. Verwendung des Heterokonjugates nach Anspruch 1 oder 2 zur Herstellung eines pharmazeutischen Mittels zur Regulation der Funktion von T-Lymphozyten und/oder B-Lymphozyten.

21. Verwendung nach Anspruch 20, worin wenigstens einer der Antikörper des Heterokonjugates mit dem Oberflächenantigen CD45 oder einer Isoform des CD45 Antigens reagiert.

22. In vitro-Verfahren zur Behandlung von Erkrankungen, umfassend die Aktivierung von T-Lymphozyten in vitro, indem man die Lymphozyten mit wenigstens einem Heterokonjugat gemäß Anspruch 1 in Kontakt bringt.

23. Verfahren nach Anspruch 22, wobei die Erkrankung ausgewählt ist unter einer infektiösen Erkrankung, Krebs, AIDS und einer Autoimmunerkrankung.

24. Pharmazeutisch verträgliche Zusammensetzung zur Behandlung einer Erkrankung, umfassend eine pharmazeutisch wirksame Menge wenigstens eines Heterokonjugates nach einem der Ansprüche 1 bis 18.

25. Verwendung wenigstens eines Heterokonjugates nach einem der Ansprüche 1 bis 18 zur Herstellung eines pharmazeutischen Mittels zur Krankheitsbehandlung, insbesondere von einer infektiösen Erkrankung, Krebs, AIDS und einer Autoimmunerkrankung.

26. In Vitro-Verwendung wenigstens eines Heterokonjugates nach einem der Ansprüche 1 bis 18 zur Diagnostizierung eines Defekts der CD-Rezeptorfunktion in einem Patienten, der an einer Erkrankung leided, die eine T-Zellfehlregulation oder -dysfunktion beinhaltet, wobei man, die Calcium-Mobilisierungsaktivität des Heterokonjugates auf die T-Lymphozyten in dem Patienten im Vergleich zur Aktivität der unkonjugierten Antikörper, welche die Komponenten des Heterokonjugates darstellen, bestimmt.

27. Verwendung eines Heterokonjugates nach Anspruch 7 oder 11 zur Herstellung eines pharmazeutischen Mittels zur selektiven Regulation von T- und B-Lymphozyten-Subpopulationen bei der Behandlung einer Autoimmunerkrankung.

28. Verfahren zur Herstellung von Heterokonjugaten gemäß Anspruch 1, brauchbar zur Regulation von Lymphozyten, umfassend die Quervernetzung wenigstens zweier Moleküle, die zur Bindung an Antigen auf Lymphozyten befähigt sind, wobei jedes Molekül mit einem anderen Antigen auf der Oberfläche einzelner Lymphozyten reagiert.

29. Verfahren nach Anspruch 28, worin die Lymphozyten, mit denen die Moleküle reagieren T-Lymphozyten sind.

30. Verfahren nach Anspruch 28, worin das Antigen ein CD-Antigen auf der Oberfläche eines T-Lymphozyten ist.

31. Verfahren nach Anspruch 28, worin das Antigen ein CD-Antigen auf der Oberfläche eines T-Lymphozyten ist.

32. Verfahren nach Anspruch 28, worin eines der Moleküle des Heterokonjugates mit dem T-Zellrezeptor oder dem assoziierten CD3 Antigen davon reagiert.

33. Verfahren nach Anspruch 28, worin eines der Moleküle des Heterokonjugates mit dem T-Zelloberflächenantigen CD5 reagiert.

34. Verfahren nach Anspruch 28 oder 29, worin die Moleküle der mit dem CD3-Antigen reagierende Antikörper und der mit dem CD4-Antigen reagierende Antikörper sind.

35. Verfahren nach Anspruch 34, worin die Antikörper den Antikörper G19-4 bedeuten, der mit dem Antikörper G17-2 quervernetzt ist.

36. Verfahren nach Anspruch 28, worin die Moleküle ein mit dem CD3-Antigen reagierender Antikörper und ein Antikörper, ausgewählt unter Antikörpern, die mit dem Antigen CD6, CD7 und CD8 reagieren, sind.

37. Verfahren nach Anspruch 28, worin die Moleküle der mit dem CD5-Antigen reagierende Antikörper und ein Antikörper, ausgewählt unter Antikörpern, die mit dem Antigen CD4, CD6 und CD8 reagieren, sind.

38. Verfahren nach Anspruch 28, worin eines der Moleküle mit dem CD45-Antigen oder Isoformen des CD45 Antigens reagiert.

39. Verfahren nach Anspruch 28, worin eines der Moleküle ein Antikörper ist, ausgewählt unter Antikörpern, die mit dem Antigen CD2, CD3, CD4 und CD28 reagieren und das andere Molekül ein mit dem CD45-reagierender Antikörper ist.

40. Verfahren nach Anspruch 28, worin eines der Moleküle ein Antikörper ist, ausgewählt unter Antikörpern, die mit dem Antigen CD2, CD3, CD4 und CD28 reagieren und das andere Molekül ein mit dem CD45R-reagierender Antikörper ist.

41. Verfahren nach einem der Ansprüche 28-40, worin die Moleküle monoklonale Antikörper sind.

42. Verfahren nach Anspruch 28, worin die Moleküle Antikörperfragmente umfassen, ausgewählt unter Fab- und $F(ab')_2$-Fragmente.

43. Verfahren nach Anspruch 28, worin die Moleküle chimäre Antikörper sind.

44. Verfahren nach Anspruch 28, worin die Quervernetzung eine chemische Quervernetzung ist.

**45.** Verfahren nach Anspruch 44, worin die chemische Quervernetzung unter Verwendung eines Reagens erfolgt, das ausgewählt ist unter Maleimidobutryloxysuccinimid und N-Succinimidyl 3-(2-pyridyldithio)propionat.

**46.** In Vitro-Verfahren zur Diagnostizierung eines Defekts in der CD-Rezeptorfunktion eines Patienten, der an einer Erkrankung leided, welche eine T-Zellfehlregulation oder -dysfunktion beinhaltet, umfassend die Bestimmung der Calciummobilisierungsaktivität eines Heterokonjugates nach einem der Ansprüche 1 oder 2 auf T-Lymphozyten des Patienten im Vergleich zur Aktivität der unkonjugierten Antikörper, welche die Komponenten des Heterokonjugates sind.

**Patentansprüche für folgenden Vertragsstaat : ES**

**1.** Verfahren zur Herstellung eines Heterokonjugates zur Regulation von Lymphozyten, umfassend wenigstens zwei Moleküle, ausgewählt unter Antikörpern und Peptidliganden, welche zur Bindung an Antigenen auf Lymphozyten befähigt sind, wobei die Moleküle miteinander quervernetzt sind und jedes Molekül mit einem anderen Antigen auf der Oberfläche einzelner Lymphozyten reagiert, wobei man diese Moleküle miteinander quervernetzt.

**2.** Verfahren nach Anspruch 1, wobei die Lymphozyten, mit denen die Moleküle reagieren, T-Lymphozyten sind.

**3.** Verfahren nach Anspruch 1 oder 2, worin das Heterokonjugat ein Antikörper-Heterokonjugat zur Aktivierung von T-Lymphozyten ist, das wenigstens zwei Antikörpermoleküle umfaßt, die miteinander quervernetzt sind, wobei jeder Antikörper mit einem anderen Antigen auf der Oberfläche von T-Lymphozyten reagiert.

**4.** Verfahren nach Anspruch 1, 2 oder 3, worin das Antigen ein CD-Antigen auf der Oberfläche eines T-Lymphozyten ist.

**5.** Verfahren nach Anspruch 1, worin das Antigen ein CD-Antigen auf der Oberfläche eines B-Lymphozyten ist.

**6.** Verfahren nach Anspruch 1, 2 oder 3, worin eines der Moleküle des Heterokonjugates mit dem T-Zellrezeptor oder dem assoziierten CD3-Antigen davon reagiert.

**7.** Verfahren nach Anspruch 1, 2 oder 3, worin eines der Moleküle des Heterokonjugates mit dem T-Zelloberflächenantigen CD5 reagiert.

**8.** Verfahren nach Anspruch 6, wobei man das Antikörperheterokonjugat CD3/CD4 erhält.

**9.** Verfahren nach Anspruch 8, wobei man das Antikörperheterokonjugat G19-4/G17-2 erhält.

**10.** Verfahren nach Anspruch 6, wobei man ein Antikörperheterokonjugat, ausgewählt unter CD3/CD2, CD3/CD6, CD3/CD7 und CD3/CD8 erhält.

**11.** Verfahren nach Anspruch 7, wobei man ein Antikörperheterokonjugat, ausgewählt unter CD5/CD4, CD5/CD6 und CD5/CD8 erhält.

**12.** Verfahren nach Anspruch 1, 2 oder 3, wobei eines der Moleküle des Heterokonjugates mit dem CD45 Antigen oder Isoformen des CD45 Antigens reagiert.

**13.** Verfahren nach Anspruch 12, wobei man das Antikörperheterokonjugat CD3/CD45, erhält.

**14.** Verfahren nach Anspruch 13, wobei man das Antikörperheterokonjugat G19-4/9.4 erhält.

**15.** Verfahren nach Anspruch 12, wobei man ein Antikörperheterokonjugat, ausgewählt unter CD2/CD45, CD4/CD45 und CD28/CD45 erhält.

**16.** Verfahren nach Anspruch 12, wobei man ein Antikörperheterokonjugat, ausgewählt unter CD2/CD45R, CD3/CD45R, CD4/CD45R und CD28/CD45R erhält.

**17.** Verfahren nach einem der vorhergehenden Ansprüche, wobei die Antikörper Antikörperfragmente umfassen, die ausgewählt sind unter Fab- und F(ab')$_2$-Fragmenten.

**18.** Verfahren nach einem der vorhergehenden Ansprüche, wobei die Antikörper monoklonal sind.

**19.** Verfahren nach einem der vorhergehenden Ansprüche, wobei die Antikörper chimär sind.

**20.** Verfahren nach einem der vorhergehenden Ansprüche, worin die chemische Quervernetzung unter Verwendung eines Reagens erfolgt, das ausgewählt ist unter Maleimidobutryloxysuccinimid und N-Succinimidyl-3-(2-pyridyl-dithio)propionat.

**21.** Verwendung wenigstens eines Heterokonjugates nach Anspruch 3 zur Herstellung eines pharmazeutischen Mittels zur Aktivierung von T-Lymphozyten.

**22.** Verwendung des Heterokonjugates nach Anspruch 1, 2 oder 3 zur Herstellung eines pharmazeutischen Mittels zur Regulation der Funktion von T-Lymphozyten und/oder B-Lymphozyten.

**23.** Verwendung nach Anspruch 22, worin wenigstens einer der Antikörper des Heterokonjugates mit dem Zelloberflächenantigen CD45 oder einer Isoform des CD45 Antigens reagiert.

**24.** In vitro-Verfahren zur Behandlung von Erkrankungen, umfassend die Aktivierung von T-Lymphozyten in vitro, indem man die Lymphozyten mit wenigstens einem Heterokonjugat gemäß Anspruch 1 in Kontakt bringt.

**25.** Verfahren nach Anspruch 24, wobei die Erkrankung ausgewählt ist unter einer infektiösen Erkrankung, Krebs, AIDS und einer Autoimmunerkrankung.

**26.** Pharmazeutisch akzeptable Zusammensetzung zur Behandlung einer Erkrankung, umfassend eine pharmazeutisch wirksame Menge wenigstens eines Heterokonjugates nach einem der Ansprüche 1 bis 20.

**27.** Verwendung wenigstens eines Heterokonjugates nach einem der Ansprüche 1 bis 20 zur Herstellung eines pharmazeutischen Mittels zur Behandlung einer Erkrankung, insbesondere von einer infektiösen Erkrankung, Krebs, AIDS oder einer Autoimmunerkrankung.

**28.** In vitro-Verwendung wenigstens eines Heterokonjugates nach einem der Ansprüche 1 bis 20 zur Diagnostizierung eines Defekts der CD-Rezeptorfunktion in einem Patienten, der an einer Erkrankung leidet, die eine T-Zellfehlregulation oder -dysfunktion beinhaltet, wobei man die Calcium-Mobilisierungsaktivität des Heterokonjugates auf die T-Lymphozyten des Patienten im Vergleich zur Aktivität der unkonjugierten Antikörper, welche die Komponenten des Heterokonjugates darstellen, bestimmt.

**29.** Verwendung eines Heterokonjugates nach Anspruch 8 oder 12 zur Herstellung eines pharmazeutischen Mittels zur selektiven Regulation von T- und B-Lymphozyten-Subpopulationen bei der Behandlung einer Autoimmunerkrankung.

**30.** In vitro-Verfahren zur Diagnostizierung eines Defekts in der CD-Rezeptorfunktion eines Patienten, der an einer Erkrankung leidet, welche eine T-Zellfehlregulation oder -dysfunktion beinhaltet, umfassend die Bestimmung der Calcium-Mobilisierungsaktivität eines Heterokonjugates nach einem der Ansprüche 1, 2 oder 3 auf T-Lymphozyten des Patienten im Vergleich zur Aktivität der unkonjugierten Antikörper, welche die Komponenten des Heterokonjugates sind.

**Patentansprüche für folgenden Vertragsstaat : GR**

**1.** Heterokonjugat zur Regulation von Lymphozyten, umfassend wenigstens zwei Moleküle, ausgewählt unter Antikörpern und Peptidliganden, welche zur Bindung an Antigenen auf Lymphozyten befähigt sind, wobei die Moleküle miteinander quervernetzt sind und jedes Molekül mit einem anderen Antigen auf der Oberfläche einzelner Lymphozyten reagiert.

**2.** Heterokonjugat nach Anspruch 1, worin das Heterokonjugat ein Antikörper-Heterokonjugat zur Aktivierung von T-Lymphozyten ist, das wenigstens zwei Antikörpermoleküle umfaßt, die miteinander quervernetzt sind, wobei jeder Antikörper mit einem anderen Antigen auf der Oberfläche von T-Lymphozyten reagiert.

**3.** Heterokonjugat nach Anspruch 1 oder 2, worin das Antigen ein CD-Antigen auf der Oberfläche eines T-Lymphozyten ist.

**4.** Heterokonjugat nach Anspruch 1, worin das Antigen ein CD-Antigen auf der Oberfläche eines B-Lymphozyten ist.

5. Heterokonjugat nach Anspruch 1 oder 2, worin eines der Moleküle des Heterokonjugates mit dem T-Zellrezeptor oder dem assoziierten CD3-Antigen davon reagiert.

6. Heterokonjugat nach Anspruch 1 oder 2, worin eines der Moleküle des Heterokonjugates mit dem T-Zelloberflächenantigen CD5 reagiert.

7. Heterokonjugat nach Anspruch 5, nämlich das Antikörperheterokonjugat CD3/CD4.

8. Heterokonjugat nach Anspruch 7, nämlich das Antikörperheterokonjugat G19-4/G17-2.

9. Heterokonjugat nach Anspruch 5, nämlich ein Antikörperheterokonjugat, ausgewählt unter CD3/CD2, CD3/CD6, CD3/CD7 und CD3/CD8.

10. Heterokonjugat nach Anspruch 6, nämlich ein Antikörperheterokonjugat, ausgewählt unter CD5/CD4, CD5/CD6 und CD5/CD8.

11. Heterokonjugat nach Anspruch 1 oder 2, worin eines der Moleküle des Heterokonjugates mit dem CD45 Antigen oder Isoformen des CD45 Antigens reagiert.

12. Heterokonjugat nach Anspruch 11, nämlich das Antikörperheterokonjugat CD3/CD45.

13. Heterokonjugat nach Anspruch 12, nämlich das Antikörperheterokonjugat G19-4/9.4.

14. Heterokonjugat nach Anspruch 11, nämlich ein Antikörperheterokonjugat, ausgewählt unter CD2/CD45, CD4/ CD45 und CD28/CD45.

15. Heterokonjugat nach Anspruch 11, nämlich ein Antikörperheterokonjugat, ausgewählt unter CD2/CD45R, CD3/CD45R, CD4/CD45R und CD28/ CD45R.

16. Heterokonjugat nach Anspruch 2, worin die Antikörper Antikörperfragmente umfassen, die ausgewählt sind unter Fab- und F(ab')$_2$-Fragmenten.

17. Antikörperheterokonjugat nach Anspruch 2, worin die Antikörper monoklonal sind.

18. Heterokonjugat nach Anspruch 2, worin die Antikörper chimär sind.

19. Verwendung wenigstens eines Heterokonjugates nach Anspruch 2 zur Herstellung eines pharmazeutischen Mittels zur Aktivierung von T-Lymphozyten.

20. Verwendung des Heterokonjugates nach Anspruch 1 oder 2 zur Herstellung eines pharmazeutischen Mittels zur Regulation der Funktion von T-Lymphozyten und/oder B-Lymphozyten.

21. Verwendung nach Anspruch 20, worin wenigstens einer der Antikörper des Heterokonjugates mit dem Zelloberflächenantigen CD45 oder einer Isoform des CD45 Antigens reagiert.

22. In vitro-Verfahren zur Behandlung von Erkrankungen, umfassend die Aktivierung von T-Lymphozyten in vitro, indem man die Lymphozyten mit wenigstens einem Heterokonjugat gemäß Anspruch 1 in Kontakt bringt.

23. Verfahren nach Anspruch 22, wobei die Erkrankung ausgewählt ist unter einer infektiösen Erkrankung, Krebs, AIDS und einer Autoimmunerkrankung.

24. Verfahren zur Herstellung einer pharmazeutisch akzeptablen Zusammensetzung zur Behandlung einer Erkrankung, wobei man eine pharmazeutisch wirksame Menge wenigstens eines Heterokonjugates nach einem der Ansprüche 1 bis 18 bereitstellt.

25. Verwendung wenigstens eines Heterokonjugates nach einem der Ansprüche 1 bis 18 zur Herstellung eines pharmazeutischen Mittels zur Behandlung einer Erkrankung, insbesondere von einer infektiösen Erkrankung, Krebs, AIDS oder einer Autoimmunerkrankung.

26. In vitro-Verwendung wenigstens eines Heterokonjugates nach einem der Ansprüche 1 bis 18 zur Diagnostizierung eines Defekts der CD-Rezeptorfunktion in einem Patienten, der an einer Erkrankung leidet, die eine T-Zellfehlregulation oder -dysfunktion beinhaltet, wobei man die Calcium-Mobilisierungsaktivität des Heterokonjugates auf die T-Lymphozyten des Patienten im Vergleich zur Aktivität der unkonjugierten Antikörper, welche die Komponenten des Heterokonjugates darstellen, bestimmt.

27. Verwendung eines Heterokonjugates nach Anspruch 7 oder 11 zur Herstellung eines pharmazeutischen Mittels zur selektiven Regulation von T- und B-Lymphozyten-Subpopulationen bei der Behandlung einer Autoimmunerkrankung.

28. Verfahren zur Herstellung von Heterokonjugaten gemäß Anspruch 1, brauchbar zur Regulation von Lymphozyten, umfassend die Quervernetzung wenigstens zweier Moleküle, die zur Bindung an Antigenen auf Lymphozyten befähigt sind, wobei jedes Molekül mit einem anderen Antigen auf der Oberfläche einzelner Lymphozyten reagiert.

29. Verfahren nach Anspruch 28, worin die Lymphozyten, mit denen die Moleküle reagieren, T-Lymphozyten sind.

30. Verfahren nach Anspruch 28, worin das Antigen ein CD-Antigen auf der Oberfläche eines T-Lymphozyten ist.

31. Verfahren nach Anspruch 28, worin das Antigen ein CD-Antigen auf der Oberfläche eines B-Lymphozyten ist.

32. Verfahren nach Anspruch 28, worin eines der Moleküle des Heterokonjugates mit dem T-Zellrezeptor oder dem assoziierten CD3 Antigen davon reagiert.

33. Verfahren nach Anspruch 28, worin eines der Moleküle des Heterokonjugates mit dem T-Zelloberflächenantigen CD5 reagiert.

34. Verfahren nach Anspruch 28 oder 29, worin die Moleküle der mit dem CD3 Antigen reagierende Antikörper und der mit dem CD4 Antigen reagierende Antikörper sind.

35. Verfahren nach Anspruch 34, worin die Antikörper den Antikörper G19-4 bedeuten, der mit dem Antikörper G17-2 quervernetzt ist.

36. Verfahren nach Anspruch 28, worin die Moleküle ein mit dem CD3 Antigen reagierender Antikörper und ein Antikörper, ausgewählt unter Antikörpern, die mit dem Antigen CD6, CD7 und CD8 reagieren, sind.

37. Verfahren nach Anspruch 28, worin die Moleküle der mit dem CD5 Antigen reagierende Antikörper und ein Antikörper, ausgewählt unter Antikörpern, die mit dem Antigen CD4, CD6 und CD8 reagieren, sind.

38. Verfahren nach Anspruch 28, worin eines der Moleküle mit dem CD45 Antigen oder Isoformen des CD45 Antigens reagiert.

39. Verfahren nach Anspruch 28, worin eines der Moleküle ein Antikörper ist, ausgewählt unter Antikörpern, die mit den Antigenen CD2, CD3, CD4 und CD28 reagieren, und das andere Molekül ein mit dem CD45 Antigen reagierender Antikörper ist.

40. Verfahren nach Anspruch 28, worin eines der Moleküle ein Antikörper ist, ausgewählt unter Antikörpern, die mit den Antigenen CD2, CD3, CD4 und CD28 reagieren, und das andere Molekül ein mit dem CD45R Antigen reagierender Antikörper ist.

41. Verfahren nach einem der Ansprüche 28-40, worin die Moleküle monoklonale Antikörper sind.

42. Verfahren nach Anspruch 28, worin die Moleküle Antikörperfragmente umfassen, ausgewählt unter Fab- und F(ab')$_2$-Fragmente.

43. Verfahren nach Anspruch 28, worin die Moleküle chimäre Antikörper sind.

44. Verfahren nach Anspruch 28, worin die Quervernetzung eine chemische Quervernetzung ist.

**45.** Verfahren nach Anspruch 44, worin die chemische Quervernetzung unter Verwendung eines Reagens erfolgt, das ausgewählt ist unter Maleimidobutryloxysuccinimid und N-Succinimidyl-3-(2-pyridyldithio)propionat.

**46.** In vitro-Verfahren zur Diagnostizierung eines Defekts in der CD-Rezeptorfunktion eines Patienten, der an einer Erkrankung leidet, welche eine T-Zellfehlregulation oder -dysfunktion beinhaltet, umfassend die Bestimmung der Calcium-Mobilisierungsaktivität eines Heterokonjugates nach einem der Ansprüche 1 oder 2 auf T-Lymphozyten des Patienten im Vergleich zur Aktivität der unkonjugierten Antikörper, welche die Komponenten des Heterokonjugates sind.

**Revendications**

**Revendications pour les Etats contractants suivants : AT, BE, CH, DE, FR, GB, IT, LI, LU, NL, SE**

**1.** Hétéroconjugué utile dans la régulation des lymphocytes comprenant au moins deux molécules sélectionnées parmi des anticorps et des peptides ligands capables de se lier à des antigènes sur les lymphocytes, lesdites molécules étant réticulées l'une à l'autre, chaque molécule étant réactive avec un antigène différent placé à la surface des lymphocytes individuels.

**2.** Hétéroconjugué de la revendication 1, où l'hétéroconjugué est un hétéroconjugué d'anticorps utile dans l'activation des lymphocytes T comprenant au moins deux molécules d'anticorps réticulées l'une à l'autre, chaque anticorps étant réactif avec un antigène différent placé à la surface des lymphocytes T.

**3.** Hétéroconjugué de la revendication 1 ou 2 où l'antigène est un antigène CD à la surface d'un lymphocyte T.

**4.** Hétéroconjugué de la revendication 1 où l'antigène est un antigène CD à la surface d'un lymphocyte B.

**5.** Hétéroconjugué de la revendication 1 ou 2 où l'une des molécules de l'hétéroconjugué est réactive avec le récepteur des cellules T ou son antigène CD3 associé.

**6.** Hétéroconjugué de la revendication 1 ou 2 où l'une des molécules de l'hétéroconjugué est réactive avec l'antigène de surface dos cellules T CD5.

**7.** Hétéroconjugué de la revendication 5, qui est un hétéroconjugué d'anticorps à CD3/CD4.

**8.** Hétéroconjugué de la revendication 7 qui est un hétéroconjugué d'anticorps G19-4/G17-2.

**9.** Hétéroconjugué de la revendication 5 qui est un hétéroconjugué d'anticorps sélectionné dans le groupe consistant en CD3/CD2, CD3/CD6, CD3/CD7 et CD3/CD8.

**10.** Hétéroconjugué de la revendication 6 qui est un hétéroconjugué d'anticorps sélectionné dans le groupe consistant en CD5/CD4, CD5/CD6 et CD5/CD8.

**11.** Hétéroconjugué de la revendication 1 ou 2, où l'une des molécules de l'hétéroconjugué est réactive avec l'antigène CD45 ou les isoformes de l'antigène CD45.

**12.** Hétéroconjugué de la revendication 11 qui est l'hétéroconjugué d'anticorps CD3/CD45.

**13.** Hétéroconjugué de la revendication 12 qui est l'hétéroconjugué d'anticorps G19-4/9.4.

**14.** Hétéroconjugué de la revendication 11 qui est un hétéroconjugué d'anticorps sélectionné dans le groupe consistant en CD2/CD45, CD4/CD45 et CD28/CD45.

**15.** Hétéroconjugué de la revendication 11 qui est un hétéroconjugué d'anticorps sélectionné dans le groupe consistant an CD2/CD45R, CD3/CD45R, CD4/CD45 et CD28/CD45R.

**16.** Hétéroconjugué de la revendication 2 où les anticorps se composent de fragments d'anticorps sélectionnés dans le groupe consistant en fragments Fab et F(ab')$_2$.

**17.** Hétéroconjugué d'anticorps de la revendication 2 où les anticorps sont monoclonaux.

EP 0 336 379 B1

**18.** Hétéroconjugué de la revendication 2 où les anticorps sont chimériques.

**19.** Utilisation d'au moins un hétéroconjugué selon la revendication 2 pour la préparation d'une composition pharmaceutique pour activer les lymphocytes T.

**20.** Utilisation de l'hétéroconjugué de la revendication 1 ou 2 pour la préparation d'une composition pharmaceutique pour la régulation de la fonction de lymphocytes T et/ou de lymphocytes B.

**21.** Utilisation selon la revendication 20 où au moins l'un des anticorps de l'hétéroconjugué est réactif avec l'antigène de surface de cellule CD45 ou une isoforme de l'antigène CD45.

**22.** Méthode in vitro pour le traitement d'une maladie comprenant l'étape d'activer les lymphocytes T in vitro par mise en contact des lymphocytes avec au moins un hétéroconjugué selon la revendication 1.

**23.** Méthode de la revendication 22 où la maladie est sélectionnée dans le groupe consistant en maladie infectieuse, cancer, SIDA et maladie auto-immune.

**24.** Composition pharmaceutiquement acceptable utile dans le traitement d'une maladie qui comprend une quantité pharmaceutiquement efficace d'au moins un hétéroconjugué selon l'une quelconque des revendications 1 à 18.

**25.** Utilisation d'au moins un hétéroconjugué selon l'une quelconque des revendications 1 à 18 pour la préparation d'une composition pharmaceutique pour le traitement d'une maladie, en particulier une maladie infectieuse, du cancer, du SIDA ou d'une maladie auto-immune.

**26.** Utilisation in vitro d'au moins un hétéroconjugué selon l'une quelconque des revendications 1 à 18 pour le diagnostic d'un défaut de la fonction de récepteur de CD chez des patients souffrent d'une maladie impliquant un dérèglement ou un disfonctionnement des cellules T, comprenant l'étape de mesurer l'activité de mobilisation du calcium de l'hétéroconjugué sur les lymphocytes T de ce patient en comparaison avec l'activité des anticorps non conjugués qui forment les composants de l'hétéroconjugué.

**27.** Utilisation d'un hétéroconjugué de la revendication 7 ou 11 pour la préparation d'une composition pharmaceutique pour réguler sélectivement les sous-populations des lymphocytes T et B dans le traitement d'une maladie auto-immune.

**28.** Méthode de préparation d'hétéroconjugués de la revendication 1 utiles dans la régulation des lymphocytes, consistant à réticuler au moins deux molécules capables de se lier à des antigènes sur des lymphocytes, chaque molécule étant réactive avec un antigène différent à la surface de lymphocytes individuels.

**29.** Méthode de la revendication 28 où lesdits lymphocytes avec lesquels lesdites molécules sont réactives sont des lymphocytes T.

**30.** Méthode de la revendication 28 où l'antigène est un antigène CD sur la surface d'un lymphocyte T.

**31.** Méthode de la revendication 28 où l'antigène est un antigène CD sur la surface d'un lymphocyte B.

**32.** Méthode de la revendication 28 où l'une des molécules de l'hétéroconjugué est réactive avec le récepteur de cellule T ou son antigène CD3 associé.

**33.** Méthode de la revendication 28 où l'une des molécules de l'hétéroconjugué est réactive avec l'antigène de surface de cellule T CD5.

**34.** Méthode de la revendication 28 ou 29 où lesdites molécules sont l'anticorps réactif avec l'antigène CD3 et l'anticorps réactif avec l'antigène CD4.

**35.** Méthode de la revendication 34 où lesdits anticorps sont l'anticorps G19-4 réticulé à l'anticorps G17-2.

**36.** Méthode de la revendication 28 où lesdites molécules sont un anticorps réactif avec l'antigène CD3 et un anticorps sélectionné dans le groupe consistant en l'anticorps réactif avec l'antigène CD6, CD7 et CD8.

**37.** Méthode de la revendication 28 où lesdites molécules sont un anticorps réactif avec l'antigène CD5 et un anticorps sélectionné dans le groupe consistant en l'anticorps réactif avec l'antigène CD4, CD6 et CD8.

**38.** Méthode de la revendication 28 où l'une des molécules est réactive avec l'antigène CD45 ou des isoformes de l'antigène CD45.

**39.** Méthode de la revendication 28 ou l'une des molécules est un anticorps sélectionné dans le groupe consistant en l'anticorps réactif avec l'antigène CD2, CD3, CD4 et CD28 et l'autre molécule est un anticorps réactif avec l'antigène CD45.

**40.** Méthode de la revendication 28 ou l'une des molécules est un anticorps sélectionné dans le groupe consistant en anticorps réactif avec l'antigène CD2, CD3, CD4 et CD28 et l'autre molécule est un anticorps réactif avec l'antigène CD45R.

**41.** Méthode selon l'une quelconque des revendications 28-40 où lesdites molécules sont des anticorps monoclonaux.

**42.** Méthode de la revendication 28 où les molécules se composent de fragments d'anticorps sélectionnés dans le groupe consistant en fragments Fab et $F(ab')_2$.

**43.** Méthode de la revendication 28 où les molécules sont des anticorps chimériques.

**44.** Méthode de la revendication 28 où ladite réticulation est une réticulation chimique.

**45.** Méthode de la revendication 44 où ladite réticulation chimique est accomplie en utilisant un réactif sélectionné dans le groupe consistant en maléimidobutyryloxy succinimide et N-succinimidyl 3-(2-pyridyldithio)propionate.

**46.** Méthode in vitro pour le diagnostic d'un défaut de la fonction de récepteur CD chez des patients souffrant d'une maladie impliquant un dérèglement ou un disfonctionnement des cellules T comprenant l'étape de mesurer l'activité de mobilisation du calcium d'un hétéroconjugué selon l'une quelconque des revendications 1 ou 2 sur les lymphocytes T de ce patient en comparaison avec l'activité des anticorps non conjugués qui forment les composants de l'hétéroconjugué.

**Revendications pour l'Etat contractant suivant : ES**

**1.** Méthode de préparation d'un hétéroconjugué utile dans la régulation des lymphocytes comprenant au moins deux molécules sélectionnées parmi des anticorps et des peptides ligands capables de se lier à des antigènes sur les lymphocytes, chaque molécule étant réactive avec un antigène différent placé à la surface de lymphocytes individuels, laquelle méthode comprend la réticulation desdites molécules l'une à l'autre.

**2.** Méthode de la revendication 1 où lesdits lymphocytes avec lesquels lesdites molécules sont réactives sont des lymphocytes T.

**3.** Méthode de la revendication 1 ou 2 où l'hétéroconjugué est un hétéroconjugué d'anticorps utile dans l'activation des lymphocytes T comprenant au moins deux molécules d'anticorps réticulées l'une à l'autre, chaque anticorps étant réactif avec un antigène différent placé à la surface des lymphocytes T.

**4.** Méthode de la revendication 1, 2 ou 3 où l'antigène est un antigène CD à la surface d'un lymphocyte T.

**5.** Méthode de la revendication 1 où l'antigène est un antigène CD à la surface d'un lymphocyte B.

**6.** Méthode de la revendication 1, 2 ou 3 où l'une des molécules de l'hétéroconjugué est réactive avec le récepteur de cellules T ou son antigène CD3 associé.

**7.** Méthode de la revendication 1, 2 ou 3 où l'une des molécules de l'hétéroconjugué est réactive avec l'antigène de surface de cellules T CD5.

**8.** Méthode de la revendication 6 où on prépare un hétéroconjugué d'anticorps CD3/CD4.

**9.** Méthode de la revendication 8 où on prépare un hétéroconjugué d'anticorps G19-4/G17-2.

EP 0 336 379 B1

**10.** Méthode de la revendication 6 où on prépare un hétéroconjugué d'anticorps sélectionné dans le groupe consistant en CD3/CD2, CD3/CD6, CD3/CD7 et CD3/CD8.

**11.** Méthode de la revendication 7 où on prépare un hétéroconjugué d'anticorps sélectionné dans le groupe consistant en CD5/CD4, CD5/CD6 et CD5/CD8.

**12.** Méthode de la revendication 1, 2 ou 3 où l'une des molécules de l'hétéroconjugué est réactive avec l'antigène CD45 ou des isoformes de l'antigène CD45.

**13.** Méthode de la revendication 12 où on prépare l'hétéroconjugué d'anticorps CD3/CD45.

**14.** Méthode de la revendication 13 où on prépare l'hétéroconjugué d'anticorps G19-4/9.4.

**15.** Méthode de la revendication 12 où on prépare un hétéroconjugué d'anticorps sélectionné dans le groupe consistant en CD2/CD45, CD4/CD45 et CD28/CD45.

**16.** Méthode de la revendication 12 où on prépare un hétéroconjugué d'anticorps sélectionné dans le groupe consistant en CD2/CD45R, CD3/CD45R, CD4/CD45R et CD28/CD45R.

**17.** Méthode de l'une quelconque des revendications précédentes où les anticorps se composent de fragments d'anticorps sélectionnés dans le groupe consistant en fragments Fab et F(ab')$_2$.

**18.** Méthode selon l'une quelconque des revendications précédentes où les anticorps sont monoclonaux.

**19.** Méthode selon l'une quelconque des revendications précédentes où les anticorps sont chimériques.

**20.** Méthode selon l'une quelconque des revendications précédentes où ladite réticulation chimique est accomplie en utilisant un réactif sélectionné dans la groupe consistant en maléimidobutyryloxy succinimide et N-succinimidyl 3-(2-pyridyldithio)-propionate.

**21.** Utilisation d'au moins un hétéroconjugué selon la revendication 3 pour la préparation d'une composition pharmaceutique pour activer les lymphocytes T.

**22.** Utilisation de l'hétéroconjugué de la revendication 1, 2 ou 3 pour la préparation d'une composition pharmaceutique pour réguler la fonction des lymphocytes T et/ou des lymphocytes B.

**23.** Utilisation selon la revendication 22 ou au moins l'un des anticorps des hétéroconjugués est réactif avec l'antigène de surface de cellule CD45 ou un isoforme de l'antigène CD45.

**24.** Méthode in vitro pour le traitement d'une maladie comprenant l'étape d'activer les lymphocytes T in vitro par mise en contact des lymphocytes avec au moins un hétéroconjugué selon la revendication 1.

**25.** Méthode de la revendication 24 où la maladie est sélectionnée dans le groupe consistant en maladie infectieuse, cancer, SIDA et maladie auto-immune.

**26.** Méthode de préparation d'une composition pharmaceutiquement acceptable utile dans le traitement d'une maladie, laquelle méthode consiste à produire une quantité pharmaceutiquement efficace d'au moins un hétéroconjugué préparé selon l'une quelconque des revendications 1 à 20.

**27.** Utilisation d'au moins un hétéroconjugué préparé selon l'une quelconque des revendications 1 à 20 pour la préparation d'une composition pharmaceutique pour le traitement d'une maladie, en particulier d'une maladie infectieuse, du cancer, du SIDA ou d'une maladie auto-immune.

**28.** Utilisation in vitro d'au moins un hétéroconjugué préparé selon l'une quelconque des revendications 1 à 20 pour le diagnostic d'un défaut de la fonction de récepteur de CD chez des patients souffrant d'une maladie impliquant un dérèglement ou un disfonctionnement des cellules T, comprenant l'étape de mesurer l'activité de mobilisation du calcium de l'hétéroconjugué sur les lymphocytes T de ce patient en comparaison avec l'activité des anticorps non conjugués qui forment les composants de l'hétéroconjugué.

29. Utilisation d'un hétéroconjugué de la revendication 8 ou 12 pour la préparation d'une composition pharmaceutique pour réguler sélectivement des sous-populations de lymphocytes T et B dans le traitement d'une maladie auto-immune.

30. Méthode in vitro pour le diagnostic d'un défaut de la fonction de récepteur CD chez des patients souffrant d'une maladie impliquant un dérèglement ou un disfonctionnement des cellules T, comprenant l'étape de mesurer l'activité de mobilisation du calcium d'un hétéroconjugué selon l'une quelconque des revendications 1, 2 ou 3 sur les lymphocytes T de ce patient en comparaison avec l'activité des anticorps non conjugués qui forment les composants de l'hétéroconjugué.

**Revendications pour l'Etat contractant suivant : GR**

1. Hétéroconjugué utile dans la régulation des lymphocytes comprenant au moins deux molécules sélectionnées parmi des anticorps et des peptides ligands capables de se lier à des antigènes sur les lymphocytes, lesdites molécules étant réticulées l'une à l'autre, chaque molécule étant réactive avec un antigène différent placé à la surface des lymphocytes individuels.

2. Hétéroconjugué de la revendication 1, où l'hétéroconjugué est un hétéroconjugué d'anticorps utile dans l'activation des lymphocytes T comprenant au moins deux molécules d'anticorps réticulées l'une à l'autre, chaque anticorps étant réactif avec un antigène différent placé à la surface des lymphocytes T.

3. Hétéroconjugué de la revendication 1 ou 2 où l'antigène est un antigène CD à la surface d'un lymphocyte T.

4. Hétéroconjugué de la revendication 1 où l'antigène est un antigène CD à la surface d'un lymphocyte B.

5. Hétéroconjugué de la revendication 1 ou 2 où l'une des molécules de l'hétéroconjugué est réactive avec le récepteur des cellules T ou son antigène CD3 associé.

6. Hétéroconjugué de la revendication 1 ou 2 où l'une des molécules de l'hétéroconjugué est réactive avec l'antigène de surface des cellules T CD5.

7. Hétéroconjugué de la revendication 5, qui est un hétéroconjugué d'anticorps à CD3/CD4.

8. Hétéroconjugué de la revendication 7 qui est un hétéroconjugué d'anticorps G19-4/G17-2.

9. Hétéroconjugué de la revendication 5 qui est un hétéroconjugué d'anticorps sélectionné dans le groupe consistant en CD3/CD2, CD3/CD6, CD3/CD7 et CD3/CD8.

10. Hétéroconjugué de la revendication 6 qui est un hétéroconjugué d'anticorps sélectionné dans le groupe consistant an CD5/CD4, CD5/CD6 et CD5/CD8.

11. Hétéroconjugué de la revendication 1 ou 2, où l'une des molécules de l'hétéroconjugué est réactive avec l'antigène CD45 ou les isoformes de l'antigène CD45.

12. Hétéroconjugué de la revendication 11 qui est l'hétéroconjugué d'anticorps CD3/CD45.

13. Hétéroconjugué de la revendication 12 qui est l'hétéroconjugué d'anticorps G19-4/9.4.

14. Hétéroconjugué de la revendication 11 qui est un hétéroconjugué d'anticorps sélectionné dans le groupe consistant en CD2/CD45, CD4/CD45 et CD28/CD45.

15. Hétéroconjugué de la revendication 11 qui est un hétéroconjugué d'anticorps sélectionné dans le groupe consistant en CD2/CD45R, CD3/CD45R, CD4/CD45 et CD28/CD45R.

16. Hétéroconjugué de la revendication 2 où les anticorps se composent de fragments d'anticorps sélectionnés dans le groupe consistant en fragments Fab et F(ab')$_2$.

17. Hétéroconjugué d'anticorps de la revendication 2 où les anticorps sont monoclonaux.

**18.** Hétéroconjugué de la revendication 2 où les anticorps sont chimériques.

**19.** Utilisation d'au moins un hétéroconjugué selon la revendication 2 pour la préparation d'une composition pharmaceutique pour activer les lymphocytes T.

**20.** Utilisation de l'hétéroconjugué de la revendication 1 ou 2 pour la préparation d'une composition pharmaceutique pour la régulation de la fonction de lymphocytes T et/ou de lymphocytes B.

**21.** Utilisation selon la revendication 20 où au moins l'un des anticorps de l'hétéroconjugué est réactif avec l'antigène de surface de cellule CD45 ou une isoforme de l'antigène CD45.

**22.** Méthode in vitro pour le traitement d'une maladie comprenant l'étape d'activer les lymphocytes T in vitro par mise en contact des lymphocytes avec au moins un hétéroconjugué selon la revendication 1.

**23.** Méthode de la revendication 22 où la maladie est sélectionnée dans le groupe consistant en maladie infectieuse, cancer, SIDA et maladie auto-immune.

**24.** Méthode de préparation d'une composition pharmaceutiquement acceptable utile dans le traitement d'une maladie, laquelle méthode consiste à prévoir une quantité pharmaceutiquement efficace d'au moins un hétéroconjugué selon l'une quelconque des revendications 1 à 18.

**25.** Utilisation d'au moins un hétéroconjugué selon l'une quelconque des revendications 1 à 18 pour la préparation d'une composition pharmaceutique pour le traitement d'une maladie en particulier une maladie infectieuse, du cancer, du SIDA ou d'une maladie auto-immune.

**26.** Utilisation in vitro d'au moins un hétéroconjugué selon l'une quelconque des revendications 1 à 18 pour le diagnostic d'un défaut de la fonction de récepteur de CD chez des patients souffrants d'une maladie impliquant un dérèglement ou un disfonctionnement des cellules T comprenant l'étape de mesurer l'activité de mobilisation du calcium de l'hétéroconjugué sur les lymphocytes T de ce patient en comparaison avec l'activité des anticorps non conjugués qui forment les composants de l'hétéroconjugué.

**27.** Utilisation d'un hétéroconjugué de la revendication 7 ou 11 pour la préparation d'une composition pharmaceutique pour réguler sélectivement les sous-populations des lymphocytes T et B dans le traitement d'une maladie auto-immune.

**28.** Méthode de préparation d'hétéroconjugués de la revendication 1 utiles dans la régulation des lymphocytes consistant à réticuler au moins deux molécules capables de se lier à des antigènes sur des lymphocytes, chaque molécule étant réactive avec un antigène différent à la surface de lymphocytes individuels.

**29.** Méthode de la revendication 28 où lesdits lymphocytes avec lesquels lesdites molécules sont réactives sont des lymphocytes T.

**30.** Méthode de la revendication 28 où l'antigène est un antigène CD sur la surface d'un lymphocyte T.

**31.** Méthode de la revendication 28 où l'antigène est un antigène CD sur la surface d'un lymphocyte B.

**32.** Méthode de la revendication 28 où l'une des molécules de l'hétéroconjugué est réactive avec le récepteur de cellule T ou son antigène CD3 associé.

**33.** Méthode de la revendication 28 où l'une des molécules de l'hétéroconjugué est réactive avec l'antigène de surface de cellule T CD5.

**34.** Méthode de la revendication 28 ou 29 où lesdites molécules sont l'anticorps réactif avec l'antigène CD3 et l'anticorps réactif avec l'antigène CD4.

**35.** Méthode de la revendication 34 où lesdits anticorps sont l'anticorps G19-4 réticulé à l'anticorps G17-2.

**36.** Méthode de la revendication 28 où lesdites molécules sont un anticorps réactif avec l'antigène CD3 et un anticorps sélectionné dans le groupe consistant en l'anticorps réactif avec l'antigène CD6, CD7 et CD8.

**37.** Méthode de la revendication 28 où lesdites molécules sont un anticorps réactif avec l'antigène CD5 et un anticorps sélectionné dans le groupe consistant en l'anticorps réactif avec l'antigène CD4, CD6 et CD8.

**38.** Méthode de la revendication 28 où l'une des molécules est réactive avec l'antigène CD45 ou des isoformes de l'antigène CD45.

**39.** Méthode de la revendication 28 ou l'une des molécules est un anticorps sélectionné dans le groupe consistant en l'anticorps réactif avec l'antigène CD2, CD3, CD4 et CD28 et l'autre molécule est un anticorps réactif avec l'antigène CD45.

**40.** Méthode de la revendication 28 ou l'une des molécules est un anticorps sélectionné dans le groupe consistant en anticorps réactif avec l'antigène CD2, CD3, CD4 et CD28 et l'autre molécule est un anticorps réactif avec l'antigène CD45R.

**41.** Méthode selon l'une quelconque des revendications 28-40 où lesdites molécules sont des anticorps monoclonaux.

**42.** Méthode de la revendication 28 où les molécules se composent de fragments d'anticorps sélectionnés dans le groupe consistant en fragments Fab et $F(ab')_2$.

**43.** Méthode de la revendication 28 où les molécules sont des anticorps chimériques.

**44.** Méthode de la revendication 28 où ladite réticulation est une réticulation chimique.

**45.** Méthode de la revendication 44 où ladite réticulation chimique est accomplie en utilisant un réactif sélectionné dans le groupe consistant en maléimidobutryloxy succinimide et N-succinimidyl 3-(2-pyridyldithio)propionate.

**46.** Méthode in vitro pour le diagnostic d'un défaut de la fonction de récepteur CD chez des patients souffrant d'une maladie impliquant un dérèglement ou un disfonctionnement des cellules T comprenant l'étape de mesurer l'activité de mobilisation du calcium d'un hétéroconjugué selon' l'une quelconque des revendications 1 ou 2 sur les lymphocytes T de ce patient en comparaison avec l'activité des anticorps non conjugués qui forment les composants de l'hétéroconjugué.

FIG.IA

FIG.IB

41

FIG. 2

# FIG. 3

FIG. 4A

FIG. 4B

FIG. 4C

FIG.5A

FIG.5B

# FIG.6

# FIG.7

# FIG. 8

# FIG.9A

# FIG.9B

# FIG. 10